# EUROPEAN PATENT APPLICATION

(11) **EP 2 752 206 A1**
(43) Date of publication of application: **09.07.2014**
(21) Application number: 12826981.8
(22) Date of filing: 31.07.2012
(51) Int. Cl.: A61L 33/00, A61L 31/00, A61M 25/00

(54) **MEDICAL INSTRUMENT**

(30) Priority: 31.08.2011 JP 2011189728
(71) Applicant: Terumo Kabushiki Kaisha, Tokyo 151-0072 (JP)
(72) Inventor: KOHAMA, Hiromasa, Fujinomiya-shi Shizuoka 418-0015 (JP); SHIMURA, Kenichi, Fujinomiya-shi Shizuoka 418-0015 (JP); SAWADA, Satoshi, Fujinomiya-shi Shizuoka 418-0015 (JP)
(74) Representative: Casalonga
(86) International application number: PCT/JP2012/069470
(87) International publication number: WO 2013/031459

(57) **Abstract**

The present invention provides a medical device of which the surface exhibits lubricity and/or antithrombotic when being wet. The medical device of the present invention includes a base material layer, and a hydrophilic layer that is supported on at least a portion of the base material layer,in which the hydrophilic layer is obtained by forming a crosslinked structure by means of reacting a compound having plural thiol groups in a molecule with a compound having reactive functional groups binding to the thiol group and hydrophilic groups in a molecule, and the reactive functional groups are thiol groups, alkenyl groups, acryl groups, or methacryl groups.

## Description

### Technical Field

The present invention relates to a medical device of which the surface exhibits lubricity and/or antithrombotic when being wet.

### Background Art

Medical devices to be inserted into the living body, such as catheters, guide wires, and indwelling needles, are required to exhibit excellent antithrombotic or lubricity so as to reduce the damage of tissues such as blood vessels and improve operability for an operator. Accordingly, a method of covering the surface of a base material with a hydrophilic polymer having antithrombotic or lubricity has been developed and put to practical use. In such medical devices, elution or peeling-off of the hydrophilic polymer from the surface of the base material causes problems in maintaining safety or operability.

In order to prevent such problems, Patent Literature 1 discloses a medical device which is obtained by preparing a polymer solution by means of dissolving a hydrophilic polymer in a solvent that swells a base material of the medical device, dipping the base material of the medical device into the polymer solution to swell the base material, and causing the polymer to be crosslinked or polymerized on the surface of the base material so as to form a surface lubricating layer on the surface of the base material.

With the method described in Patent literature 1, the surface lubricating layer can be firmly fixed to the base material to some extent.

Particularly, when the base material itself swells by the hydrophobic polymer solution, the base material and the hydrophilic polymer forming the surface lubricating layer form an interpenetrating network structure, whereby the surface lubricating layer can be extremely firmly fixed. On the other hand, when the base material does not easily swell by the hydrophilic polymer solution, the hydrophilic polymer forming the surface lubricating layer is fixed to the base material by only an insolubilization effect produced by crosslinking or polymerization. Accordingly, compared to the base material forming an interpenetrating network structure, the risk of peeling off of the surface lubricating layer becomes higher. Therefore, a coating method that enables the hydrophilic polymer to be more firmly fixed onto the surface of a poorly swellable base material is required.

Regarding a method of firmly fixing a large amount of hydrophilic polymer onto the surface of polymeric base material, Patent Literature 2 discloses a medical device in which a polymer material forming a base material layer has a first higher-order structure in the molecule thereof and a first functional group disposed in at least one of the terminals of the first higher-order structure, and a hydrophilic polymer has a second higher-order structure, which can interact with the first higher-order structure, in the molecule thereof and a second functional group which is disposed in at least one of the terminals of the second higher-order structure and can form a hydrogen bond with the first functional group.

Moreover, as a method of fixing a hydrophilic polymer to a metallic base material, Patent Literature 3 discloses a method of forming an middle layer by means of bonding a compound which has plural thiol groups in the molecule thereof to the surface of the metallic base material, then forming a surface lubricating layer by reacting epoxy groups or isocyanate groups as reactive functional groups of the hydrophilic polymer with residual thiol groups of the middle layer so as to bond (fix) the surface lubricating layer to the base material via the middle layer.

### Citation List

### Patent Literature

Patent Literature 1: JP-A-8-33704
Patent Literature 2: JP-A-2007-289299
Patent Literature 3: US 2011/0274918 A

### Summary of Invention

However, the method disclosed in Patent Literature 2 has a problem that there is a limitation on the combination of the polymeric base material and the hydrophilic polymer.

Moreover, the method of Patent Literature 3 has problems that it takes a time for forming the surface lubricating layer (fixing the hydrophilic polymer); epoxy groups or isocyanate groups as reactive functional groups of the hydrophilic polymer are highly reactive, and the hydrophilic polymer exhibits poor stability when being in a state of solution, hence the surface lubricating layer needs to be formed (hydrophilic polymer needs to be fixed) promptly after the solution of the hydrophilic polymer is prepared; and the like. Therefore, this method is not preferable from an industrial view point such as mass production. Furthermore, epoxy group or isocyanate groups as reactive functional groups of the hydrophobic polymer are easily influenced by water, and if a water-containing solvent is used, the formed surface lubricating layer is easily peeled off. Accordingly, in selecting or managing the solvent to be used, careful manner such as preparing the solution by means of dissolving the hydrophilic polymer in a solvent not containing water and decreasing humidity in advance at the time of forming the middle layer or surface lubricating layer is required.

The present invention has been made in consideration of the above circumstances and provides a medical device in which a base material layer is firmly fixed by a simple method to a hydrophilic layer that imparts lubricity and/or antithrombotic to the surface when being wet and which permanently exhibits excellent surface lubricity and/or antithrombotic when being used.

The present invention for achieving the above object is (1) a medical device including a base material layer; and a hydrophilic layer that is supported on at least a portion of the base material layer, in which the hydrophilic layer is obtained by forming a crosslinked structure by means of reacting a compound having plural thiol groups in a molecule with a compound having reactive functional groups binding to the thiol group and hydrophilic groups in a molecule, and the reactive functional groups are thiol groups, alkenyl groups, acryl groups, or methacryl groups.

The present invention for achieving the above object is the medical device according to (1), in which (2) the hydrophilic groups are polyoxyethylene groups, hydroxyl groups, carboxyl group, amide group, sulfonic acid groups, or salts of these.

The present invention for achieving the above object is the medical device according to (1) or (2), in which (3) the hydrophilic layer is obtained by coating the base material layer with a mixed solution formed of a single solution that is obtained by dissolving the compound having plural thiol groups in a molecule and the compound having the reactive functional groups and hydrophilic groups in a solvent, and then reacting the thiol groups with the reactive functional groups.

### Brief Description of Drawings

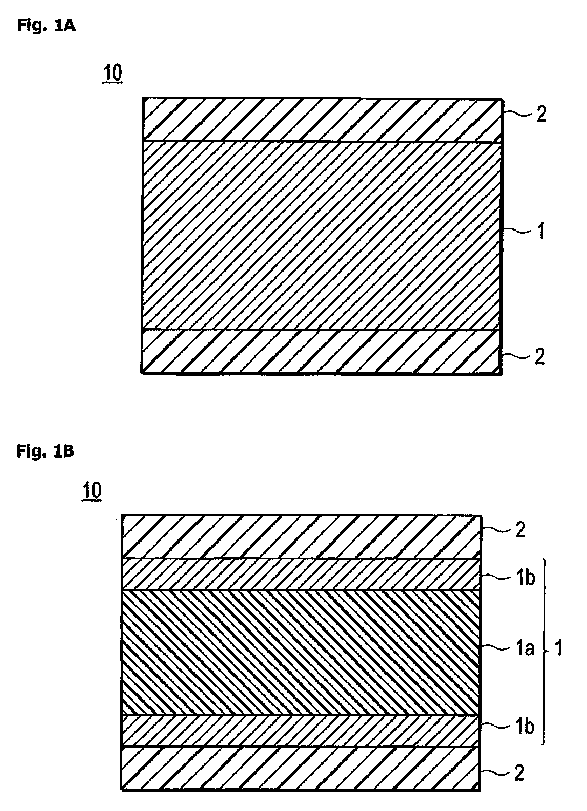

[Fig. 1A] Fig. 1A is a partial cross-sectional view schematically illustrating the constitution of layers laminated on the surface of an embodiment of the medical device according to of the present invention of which the surface exhibits lubricity and/or antithrombotic when being wet (hereinafter, simply abbreviated to a "medical device"). In Fig. 1A, 1 indicates a base material layer, 2 indicates a hydrophilic layer, and 10 indicates a medical device respectively.
[Fig. 1B] Fig. 1B is a partial cross-sectional view schematically illustrating a constitution example showing different constitution of layers laminated on the surface, as an application example of the present embodiment. In Fig. 1B, 1 indicates a base material layer, 1a indicates a core portion of the base material layer, 1b indicates a surface layer, 2 indicates a hydrophilic layer, and 10 indicates a medical device respectively.

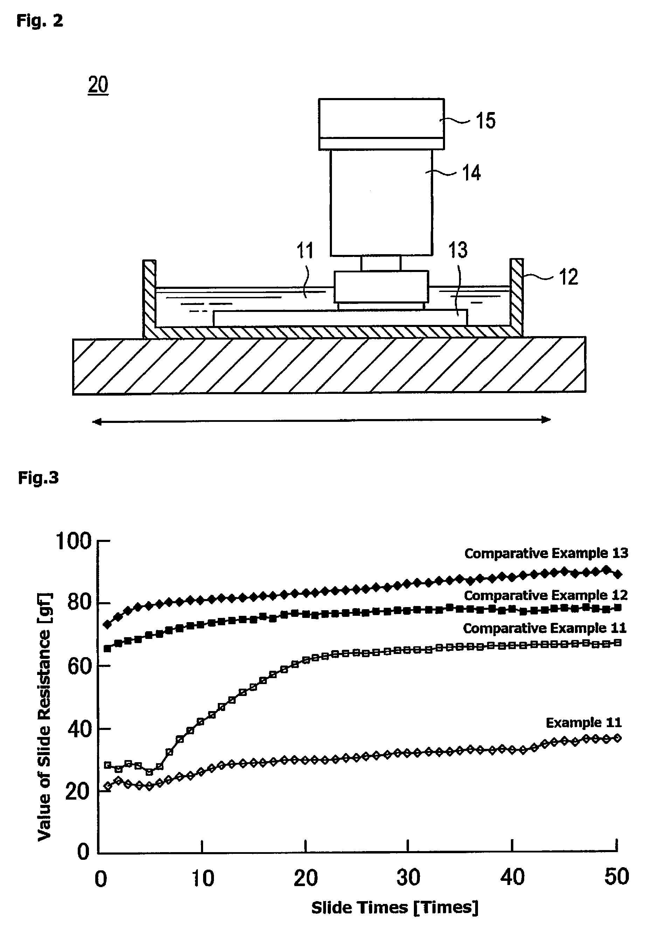

[Fig. 2] Fig. 2 is a schematic view of an apparatus (friction measuring apparatus) for evaluating surface lubricity retention that is used in examples. In Fig. 2, 11 indicates water, 12 indicates a petri dish, 13 indicates a sample, 14 indicates a terminal, 15 indicates a weight, and 20 indicates a friction measuring apparatus respectively.
[Fig. 3] Fig. 3 is a view showing evaluation results of surface lubricity retention of the medical devices (samples) obtained in Example 11 and Comparative examples 11 to 13.

### Description of Embodiments

The present invention provides a medical device including a base material layer; and a hydrophilic layer supported on at least a portion of the base material layer, in which the hydrophilic layer is obtained by forming a crosslinked structure by means of reacting a compound having plural thiol groups (-SH: called mercapto or sulfhydryl groups in some cases) in a molecule (hereinafter, also simply called a "thiol compound") with a compound having reactive functional groups binding to the thiol groups (hereinafter, also simply called "reactive functional groups") and hydrophilic groups in a molecule (hereinafter, also simply called a "hydrophilic compound"), and the reactive functional groups are thiol groups, alkenyl groups, acryl groups, or methacryl groups. In the present invention, thiol groups of the thiol compound are reacted with reactive functional groups (thoiol groups, alkenyl groups, acryl groups, or methacryl groups) of the hydrophilic compound to form a crosslinked structure, and the thiol compound is bonded to the base material layer through residual thiol groups in the thiol compound, whereby the hydrophilic layer is firmly fixed to the base material layer. That is, due to the hydrophilic layer, the surface of the medical device according to the present invention exhibits lubricity and/or antithrombotic when being wet. Moreover, in the medical device according to the present invention, various base material layers are firmly fixed to various hydrophilic layers by a simple method through thiol groups of the thiol compound. As a result, the medical device can permanently exhibit excellent lubricity and/or antithrombotic when being used.

The present invention is characterized in that the hydrophilic compound that contains thiol groups, alkenyl groups, acryl groups, or methacryl groups as reactive functional groups is used. These reactive functional groups have a low degree of reactivity, and exhibit for the first time the reactivity by being subjected to specific treatment such as heating, UV irradiation, or plasma irradiation. Accordingly, the hydrophilic compound exhibits excellent stability when being in a state of solution. Therefore, it is not necessary to prepare a solution of the hydrophilic compound whenever the operation for forming the hydrophobic layer is performed, and this is preferable from the industrial view point such as ease of operation or mass production. In addition, thiol groups, alkenyl groups, acryl groups, or methacryl groups as reactive functional groups of the hydrophilic compound are hardly influenced by water. Consequently, even if a solution of the hydrophilic compound is prepared using a water-containing solvent, and the hydrophilic layer is formed of the solution, the formed hydrophilic layer can be firmly supported on (fixed to) the base material layer. Therefore, according to the present invention, it is possible to effectively inhibit and prevent the formed hydrophilic layer from being eluted or peeled off from the surface of the base material. Furthermore, a solvent can be freely selected or managed during the step of forming the hydrophilic layer, and various conditions including humidity can also be freely set during the step of forming the hydrophilic layer.

In addition to the above advantages, according to the medical device according to the present invention, the hydrophilic layer can be prepared by performing once a step of coating the base material layer with a mixed solution formed of a single solution that is obtained by dissolving the thiol compound and the hydrophilic compound in a solvent and reacting thiol groups with reactive functional groups. Moreover, since the hydrophilic compound contains thiol groups, alkenyl groups, acryl groups, or methacryl groups as reactive functional groups, and these reactive functional groups exhibit a high degree of reactivity by being subjected to treatment such as heating, UV irradiation, or plasma irradiation, it is possible to shorten the time taken for preparing the hydrophilic layer.

Hereinafter, embodiments of the medical device according to the present invention will be described with reference to attached drawings.

Fig. 1A is a partial cross-sectional view schematically illustrating the constitution of layers laminated on the surface of a first embodiment of the medical device according to the present invention of which the surface exhibits lubricity and/or antithrombotic when being wet (hereinafter, also simply called a "medical device"). Fig. 1B is a partial cross-sectional view schematically illustrating a constitution example showing different constitution of layers laminated on the surface, as an application example of the present embodiment. As shown in Figs. 1A and 1B, the medical device 10 of the present embodiment includes a base material layer 1 and a hydrophilic layer 2 that covers at least a portion of the base material layer 1 (the drawing shows an example in which the entire base material layer in the drawing is covered with the hydrophilic layer). The hydrophilic layer 2 is characterized in that this layer is obtained by forming a croslinked structure by means of reacting a thiol compound having plural thiol groups in a molecule with a compound having reactive functional groups (thiol groups, alkenyl groups, acryl groups, or methacryl groups) binding to thiol groups and hydrophilic groups in a molecule. Herein, the hydrophilic layer 2 binds to the base material layer 1 through thol groups remaining in the crosslinked structure. Particularly, it is preferable for the hydrophilic layer 2 to be obtained by coating the base material layer 1 with a mixed solution obtained by dissolving the thiol compound and the hydrophilic compound in a solvent and then reacting the thiol groups with the reactive functional groups. In Figs. 1A and 1B, the hydrophilic layer 2 is formed on both surfaces of the base material layer 1. However, the present invention is not limited thereto, and for example, the hydrophilic layer 2 may be formed only on one surface of the base material layer 1. Alternatively, the hydrophilic layer 2 may also be formed on the side surface of the base material layer 1. Moreover, the hydrophilic layer 2 may be formed in a portion of the surface or the side surface of the base material layer 1.

Hereinafter, each of the constituent members of the medical device of the present embodiment will be described in detail.

### (1) Base material layer 1

The base material layer 1 constituting the medical device 10 of the present embodiment may be constituted with any material, and there is no particular limitation on the material. Specific examples of the material constituting (forming) the base material layer 1 include metallic materials, polymer materials, glass, and the like. The whole (entire) base material layer 1 may be constituted with (formed of) any of the above materials. Alternatively, as shown in Fig. 1B, the base material layer 1 may have a structure in which the surface of a core portion 1a of the base material layer constituted with (formed of) any of the above materials may be covered (coated) with another material among the above materials by an appropriate method to constitute (form) a surface layer 1b. Examples of the structure of the latter case include a structure in which the surface of the core portion 1a of the base material layer formed of a resin material and the like is covered (coated) with a metallic material by an appropriate method (conventionally known method such as plating, metal deposition, or sputtering) to form the surface layer 1b; a structure in which the surface of the core portion 1a of the base material layer formed of hard reinforcing material such as a metallic material or a ceramic material is coated with a polymer material that is more flexible than the reinforcing material such as a metallic material by an appropriate method (conventionally known method such as dipping, spraying, or coating/printing), or the reinforcing material of the core portion 1a of the base material layer forms a complex together with the polymer material of the surface layer 1b (through an appropriate reaction treatment) to form the surface layer 1b; and the like. Accordingly, the core portion 1a of the base material layer may be a multilayer structure formed by laminating plural layers of different materials, a structure (complex) formed by connecting members together that are formed of different materials for each part of the medical device, or the like. In addition, a middle layer (not shown in the drawing) may also be formed between the core portion 1a of the base material layer and the surface layer 1b. The surface layer 1b may also be a multilayer structure formed by laminating plural layers of different materials, a structure (complex) formed by connecting members together that are formed of different materials for each part of the medical device, or the like. In Fig 1B, the surface layer 1b is formed on both surfaces of the core portion 1a of the base material layer. However, the present invention is not limited thereto, and for example, the surface layer 1b may be formed only on one surface of the core portion 1a of the base material layer. Alternatively, the surface layer 1b may be formed in a portion of the core portion 1a of the base material layer.

Among the materials constituting (forming) the base material layer 1, the metallic materials are not particularly limited, and metallic materials that are generally used for catheters, guide wires, indwelling needles are used. Specific examples thereof include various stainless steels (SUS) such as SUS304, SUS316L, SUS420J2, and SUS 630, gold, platinum, silver, copper, nickel, cobalt, titanium, iron, aluminum, tin, various alloys such as a nickel-titanium (Ni-Ti) alloy, a nickel-cobalt (Ni-Co) alloy, a cobalt-chromium (Co-Cr) alloy, and a zinc-tungsten (Zn-W) alloy, metal-ceramic complexes, and the like. One kind among the above may be used alone, or two or more kinds among the above may be used concurrently. As the above metallic material, metallic materials optimal as the base material for catheters, guide wires, indwelling needles, and the like as the use of the medical device may be appropriately selected.

Moreover, as the polymer material, polymer materials that are generally used for catheters, guide wires, indwelling needles, and the like are used without particular limitation. Specific examples thereof include polyamide resins such as Nylon 6, Nylon 11, Nylon 12, and Nylon 66 (all of which are registered trademarks), polyolefin resins including polyethylene resins such as Linear Low-Density Polyethylene (LLDPE), Low-Density Polyethylene (LDPE), High-Density Polyethylene (HDPE), and Ultra High Molecular Weight Polyethylene (UHPE or UHMWPE), or polypropylene resins, modified polyolefin resins, epoxy resins, urethane resins, diallyl phthalate resins (allyl resins), polycarbonate resins, fluororesins, amino resins (urea resins, melamine resins, and benzoguanamine resins), polyester resins, styrol resins, acrylic resins, polyacetal resins, vinyl acetate resins, phenol resins, vinyl chloride resins, silicone resins (silicon resins), polyether resins, polyimide resins, and the like. One kind among the above may be used alone, or two or more kinds among the above may be concurrently used. As the above polymer material, polymer materials optimal as the base material for catheters, guide wires, indwelling needles, and the like as the use of the medical device may be appropriately selected.

The core portion 1a of the base material layer and the surface layer 1b are not particularly limited, and specifically, the same materials as those of the base material layer 1 can be used.

Moreover, the shape of the base material layer is not particularly limited and can be appropriately selected among a sheet shape (plate), a line shape (wire), a tube shape, and the like according to the form of use.

The materials usable for the above middle layer (not shown in the drawing) are not particularly limited and can be appropriately selected among materials that can sufficiently function to bond the core portion 1a of the base material layer to the surface layer 1b. For example, the same materials as those of the base material layer 1 can be used, but the present invention is not limited to these.

### (2) Hydrophilic layer 2

The hydrophilic layer 2 constituting the medical device of the present embodiment is supported on at least a portion of the base material layer 1 (Fig. 1 shows an example in which the hydrophilic layer 2 is supported on the entire base material layer in the drawing), and contains a hydrophilic compound having reactive functional groups (thiol groups, alkenyl groups, acryl groups, or methacryl groups) and hydrophilic groups as well as a thiol compound having plural thiol groups in a molecule. The hydrophilic layer 2 is obtained by forming a crosslinked structure by means of reacting thiol groups of the thiol compound with the reactive functional groups of the hydrophilic compound. At this time, thiol groups in the thiol compound that remain in the crosslinked structure bind to the base material layer 1. As a result, the hydrophilic layer 2 covers at least a portion of the base material layer 1 (the drawing shows an example in which the hydrophilic layer covers the entire base material layer in the drawing).

The thickness of the hydrophilic layer 2 constituting the medical device of the present embodiment is not particularly limited. It is preferable for the hydrophilic layer 2 to have such a thickness that the hydrophilic layer 2 can be firmly fixed to the base material layer 1 and can permanently exhibit excellent surface lubricity and/or antithrombotic when being used. From the viewpoint described above, the thickness of the hydrophilic layer (thickness of the hydrophilic layer not yet being swelled) is within a range of 0.1 µm to 5 µm and desirably within a range of 0.5 µm to 5 µm. If the thickness is within the above range, a uniform coat can be easily formed, and the medical device can sufficiently exhibit surface lubricity and/or antithrombotic when being wet. Moreover, even when the medical device according to the present invention is inserted into a blood vessel and the like in the living body, for example, that is, even when the medical device is passed through a site (for example, the inside of a peripheral blood vessel or the like) where there is a small clearance between the blood vessel and the medical device, the medical device can be easily passed through such a site. In addition, internal tissues such as blood vessels and the like are practically not damaged or are never damaged. Furthermore, if the thickness is within the above range, even when the medical device is passed through a site (for example, the inside of a peripheral blood vessel or the like) where there is a small clearance between a blood vessel and the medical device, the hydrophilic layer is rarely or never peeled off from the base material layer. Accordingly, in view of safety, such a thickness is preferable.

The method of forming the hydrophilic layer 2 is not particularly limited. However, it is preferable to use a method of coating the base material layer with a mixed solution formed of a single solution that is obtained by dissolving the thiol compound and the hydrophilic compound in a solvent and then reacting the thiol groups with the reactive functional groups.

The reason why the hydrophilic layer 2 is supported on at least a portion of the surface of the base material layer 1 is that the whole surface (entire surface) of medical device such as catheters, guide wires, and indwelling needles as the use of the medical instrument does not need to exhibit lubricity and/or antithrombotic when being wet. The hydrophilic layer 2 may be supported only on the surface portion (a portion of the surface or the entire surface) that is required to exhibit lubricity and/or antithrombotic when being wet. The word "supported" mentioned herein refers to a state where the hydrophilic layer 2 is fixed in a state of not being easily detached from the surface of the base material layer 1.

The thiol compound is not particularly limited as long as it is a compound having plural thiol groups in a molecule. However, it is preferable for the thiol compound to have a structure in which the thiol groups remaining on the uppermost surface of the thiol compound are easily exposed such that the residual thiol groups easily bind to the base material layer 1 when the thiol compound forms a crosslinked structure by reacting with the hydrophilic compound. From the viewpoint described above, the thiol compound may be a compound having two or more thiol groups in a single molecule. However, it is preferable to use a thiol compound having 2 to 10, more preferably 2 to 6, and particularly preferably 3 to 6 thiol groups in a single molecule.

From the viewpoint described above, the thiol compound may be linear, branched, or cyclic, and preferable examples thereof include compounds having 2 thiol groups in a molecule, such as 1,2-ethanedithiol, 1,2-propanedithiol, 1,3-propanedithiol, 1,4-butanedithiol, 2,3-butanedithiol, 1,5-pentanedithiol, 1,6-hexaneditiol, 1,8-octanedithiol, 3,6-dioxa-1,8-octanedithiol, bis(2-mercaptoethyl)ether, bis(2-mercaptoethyl)sulfide, 1,2-benzenedithiol, 1,4-benzenedithiol, 1,4-bis(mercaptomethyl)benzene, toluene-3,4-dithiol, 1,5-dimercaptonaphthalene, 2,6-dimercaptopurine, 4,4'-biphenyldithiol, 4,4'-thiobisbenzenethiol, and tetraethylene glycol bis(3-mercaptopropionate); compounds having 3 thiol groups in a molecule, such as 1,3,5-benzenetrithiol, tris-[(3-mercaptopropionyloxy)-ethyl]-isocyanurate (TEMPIC), triazine trithiol, and trimethylolpropane tris(3-mercaptopropionate) (TMMP); compounds having 4 thiol groups in a molecule, such as pentaerythritol tetrakis(mercaptoacetate), pentaerythritol tetrakis(3-mercaptopropionate), and pentaerythritol tetrakis(3-mercaptobutyrate); compounds having 6 thiol groups in a molecule, such as dipentaerythritol hexakis(3-mercaptopropionate); derivatives or polymers of these; and the like. One kind among the above may be used alone, or two or more kinds among the above may be used concurrently. Compounds having 2 to 10 thiol groups, such as tetraethylene glycol bis(3-mercaptopropionate), tris-[(3-mercaptopropionyloxy)-ethyl]-isocyanurate (TEMPIC), and dipentaerythritol hexakis(3-mercaptopropionate), are preferable, and tris-[(3-mercaptopropionyloxy)-ethyl]-isocyanurate (TEMPIC) and dipentaerythritol hexakis(3-mercaptopropionate) are more preferable, in the respect that these compounds have a structure in which residual thiol groups are easily exposed on the uppermost surface such that the residual thiol groups easily bind to the base material layer 1 when these compounds form a crosslinked structure by reacting with the hydrophilic compound, have a stabilized molecular frame, and have excellent affinity with the surface of the base material layer 1. The present embodiment is not limited to the thiol compounds exemplified above, and other thiol compounds can also be used as long as the effect of the present invention can be effectively expressed.

The hydrophilic compound according to the present invention has reactive functional groups binding to the thiol groups and hydrophilic groups in a molecule. The reactive functional groups of the hydrophilic compound are thiol groups (-SH), alkenyl groups, acryl groups (CH₂=CH-COO-), or methacryl groups (CH₂=C(CH₃)-COO-). These reactive functional groups exhibit excellent stability in a solvent. When being simply mixed with thiol groups, the reactive functional groups do not react with the thiol groups. They react for the first time with the thiol group after being subjected to specific treatment such as heating treatment, UV treatment, or plasma treatment. Accordingly, the thiol compound and the hydrophilic compound can be stored in a state of a single solution obtained by dissolving these compounds in a solvent. Moreover, it is not necessary to prepare a solution containing the thiol compound and the hydrophilic compound whenever the operation for forming the hydrophilic layer is performed. Therefore, this is very preferable from the industrial viewpoint. In addition, the above reactive functional groups exhibit high reactivity by the treatment such as heating treatment, UV treatment, or plasma treatment, hence the reactive functional groups can efficiently react with and firmly fixed to the thiol compound. The hydrophilic compound may contain only one kind of the reactive functional group or contain two or more kinds thereof as a mixture. Among the above reactive functional groups, the alkenyl groups are not particularly limited as long as they can react with the thiol groups of the thiol compound. The alkenyl groups are monovalent groups (-CₙH₂ₙ₋₁) formed when one hydrogen atom is removed from any carbon atom of alken. Specifically, alkenyl groups having 2 to 10 carbon atoms are preferable, and alkenyl groups having 2 to 8 carbon atoms are more preferable. Specific examples of the alkenyl groups include a vinyl group, a 1-propenyl group, an allyl group, an isopropenyl group, a 1-butenyl group, a 2-butenyl group, a 1-pentenyl group, a 2-pentenyl group, and the like.

The hydrophilic groups of the hydrophilic compound are not particularly limited as long as they make the surface of the medical device exhibit lubricity and/or antithrombotic when being wet. Examples of the hydrophilic groups include a polyoxyethylene group [-(OCH₂CH₂)ₙ- or - (CH₂CH₂O)ₙ-; herein, n is an integer of 1 to 2,000], a hydroxyl group (-OH), a carboxyl group (-COOH), an amide group [-C(=O)N(R)(R'); herein, each of R and R' independently represents hydrogen atom, leaner or branched alkyl groups (for example, a methyl group, an ethyl group, a propyl group, an isopropyl group, a butyl group, a sec-butyl group, and a tert-butyl group) having 1 to 8 carbon atoms, a sulfonic acid group (-SO₃H) or a salt thereof (-SO₃X; herein, X is sodium or potassium), and the like. The hydrophilic layer containing the hydrophilic compound having these hydrophilic groups can exhibit surface lubricity and/or antithrombotic when being wet. Particularly, it is preferable to use hydrophilic compounds having polyoxyethylene groups as hydrophilic groups, since both the surface lubricity and antithrombotic can be simultaneously exhibited. The hydrophilic compound may contain only one kind of the hydrophilic group or contain two or more kinds thereof as a mixture.

The hydrophilic compound having the above reactive functional groups and hydrophilic groups is not particularly limited as long as it can react with thiol groups of a thiol compound and can exhibit surface lubricity and/or antithrombotic when being wet. Specific examples of the hydrophilic compound include compounds having thiol groups and polyoxyethylene groups, such as O-[2-(3-mercaptopropionylamino)ethyl]-O'-methylpolyethyleneglycol, O-[2-(3-mercaptopropionylamino)ethyl]-O'-ethylpolyethyleneglycol, O-[2-(3-mercaptobutyrylamino)ethyl]-O'-methylpolyethyleneglycol, O-[2-(3-mercaptobutyrylamino)ethyl]-O'-ethylpolyethyleneglycol, O-[2-(3-mercaptoisobutyrylamino)ethyl]-O'-methylpolyethyleneglycol, O-[2-(3-mercaptoisobutyrylamino)ethyl]-O'-ethylpolyethyleneglycol, O-[2-(3-mercaptovalerylamino)ethyl]-O'-methylpolyethyleneglycol, O-[2-(3-mercaptovalerylamino)ethyl]-O'-ethylpolyethyleneglycol, O-[2-(3-mercaptoisovalerylamino)ethyl]-O'-methylpolyethyleneglycol, O-[2-(3-mercaptoisovalerylamino)ethyl]-O'-ethylpolyethyleneglycol, mercaptopolyethyleneglycol, O-[2-(3-mercaptopropionylamino)ethyl]polyethyleneglycol, O-[2-(3-mercaptobutyrylamino)ethyl]polyethyleneglycol, O-[2-(3-mercaptoisobutyrylamino)ethyl]polyethyleneglycol, O-[2-(3-mercaptovalerylamino)ethyl]polyethyleneglycol, and O-[2-(3-mercaptoisovalerylamino)ethyl]polyethyleneglycol; compounds having thiol groups and hydroxyl groups, such as 2-mercaptoethanol, 3-mercapto-1-propanol, 3-mercapto-2-propanol, 4-mercapto-1-butanol, 4-mercapto-2-butanol, 4-mercapto-3-butanol, 1-mercapto-1,1-methanediol, 1-mercapto-1,1-ethanediol, 3-mercapto-1,2-propanediol (α-thioglycerol), 2-mercapto-1,2-propanediol, 2-mercapto-2-methyl-1,3-propanediol, 2-mercapto-2-ethyl-1,3-propanediol, 1-mercapto-2,2-propanediol, 2-mecaptoethyl-2-methyl-1,3-propanediol, and 2-mercaptoethyl-2-ethyl-1,3-propanediol; compounds having thiol groups and carboxyl groups, such as 2-mercaptoacetic acid, 2-mercaptopropionic acid, 3-mercaptopropionic acid, 3-mercaptobutanoic acid, 2-mercaptoisobutyric acid, 3-mercaptoisobutyric acid, 3-mercapto-3-methylbutyric acid, 2-mercaptovaleric acid, 3-mercaptoisovaleric acid, 4-mercaptovaleric acid, and 3-phenyl-3-mercaptopropionic acid; compounds having thiol groups and amide groups, such as 2-mercaptoacetic acid amide, N,N-dimethyl-2-mercaptoacetic acid amide, 2-mercaptopropionic acid amide, N,N-dimethyl-2-mercaptopropionic acid amide, 3-mercaptopropionic acid amide, N,N-dimethyl-3-meracptopropionic acid amide, 3-mercaptobutanoic acid amide, N,N-dimethyl-3-mercaptobutanoic acid amide, 2-mercaptoisobutyric acid amide, N,N-dimethyl-2-mercaptoisobutyric acid amide, 3-mercaptoisobutyric acid amide, N,N-dimethyl-3-mercaptoisobutyric acid amide, 3-mercapto-3-methylbutyric acid amide, N,N-dimethyl-3-mercapto-3-methylbutyric acid amide, 2-mercaptovaleric acid amide, N,N-dimethyl-2-mercaptovaleric acid amide, 3-mercaptoisovaleric acid amide, N,N-dimethyl-3-mercaptoisovaleric acid amide, 4-mercaptovaleric acid amide, N,N-dimethyl-4-mercaptovaleric acid amide, 3-phenyl-3-mercaptopropionic acid amide, and N,N-dimethyl-3-phenyl-3-mercaptopropionic acid amide; compounds having thiol groups and sulfonic acid groups (-SO₃H) or a salt thereof, such as 2-mercaptoethanesulfonic acid, sodium 2-mercaptoethanesulfonate, potassium 2-mercaptoethanesulfonate, mercaptopropanesulfonic acid, sodium mercaptopropanesulfonate, and potassium mercaptopropanesulfonate; compounds having alkenyl groups and polyoxyethylene groups, such as polyethylene glycol vinyl ether, polyethylene glycol-1-propenyl ether, polyethylene glycol allyl ether, polyethylene glycol isopropenyl ether, polyethylene glycol-1-butenyl ether, polyethylene glycol-2-butenyl ether, polyethylene glycol-1-pentenyl ether, polyethylene glycol-2-pentenyl ether, polyethylene glycol divinyl ether, and polyethylene glycol diallyl ether; compounds having alkenyl groups and hydroxyl groups, such as tetramethylene glycol monovinyl ether, ethylene glycol monovinyl ether, 3,4-dihydro-2H-pyran-2-methanol, allyl alcohol, 1-buten-3-ol, 2-buten-1-ol, ethylene glycol monoallyl ether, and 3-allyloxy-1,2-propanediol; compounds having alkenyl groups and carboxyl groups, such as acrylic acid, methacrylic acid, crotonic acid, fumaric acid, maleic acid, 3-allyloxypropionic acid, 3-butenoic acid, trans-3-hexenedioic acid, and trans-3-pentenoic acid; compounds having alkenyl groups and amide groups, such as N,N-dimethyl(meth)acrylamide, N,N-diethyl(meth)acrylamide, N,N-methylethyl(meth)acrylamide, and (meth)acrylamide; compounds having alkenyl groups and sulfonic acid groups (-SO₃H) or a salt thereof, such as allylsulfonic acid and (meth)acrylic acid-3-sulfopropyl; compounds having (meth)acryl groups and polyoxyethylene groups, such as polyethylene glycol methyl ether (meth)acrylate and polyethylene glycol ethyl ether (meth)acrylate; compounds having (meth)acryl groups and hydroxyl groups, such as hydroxymethyl (meth)acrylate, 2-hydroxyethyl (meth)acrylate, and 2-hydroxypropyl (meth)acrylate; compounds having (meth)acrylate groups and carboxyl groups, such as 6-(meth)acrylamidehexanoic acid; compounds having (meth)acryl groups and sulfonic acid groups (-SO₃H) or a salt thereof, such as 2-(meth)acrylamide-2-methylpropanesulfonic acid; and the like. The "(meth)acryl" means "acryl and/or methacryl".

The method of forming the hydrophilic layer 2 is not particularly limited. However it is preferable to use a method of coating the base material layer with a mixed solution formed of a single solution that is obtained by dissolving the thiol compound and the hydrophilic compound in a solvent and then reacting the thiol groups with the reactive functional groups.

The mixing ratio between the thiol compound and the hydrophilic compound is not particularly limited, as long as the ratio enables thiol groups of the thiol compound to bind to the reactive functional groups of the hydrophilic compound to form a crosslinked structure, and enables the hydrophilic layer to be fixed to the base material layer through the thiol compound. Moreover, the mixing ration between the thiol compound and the hydrophilic compound varies with the number of tihol groups in a molecule of the thiol compound or the amount of the reactive functional groups of the hydrophilic compound to be reacted. Generally, the thiol compound is mixed preferably in an amount of 0.1 parts by weight to 1,000,000 parts by weight, more preferably in an amount of 0.5 parst by weight to 100,000 parts by weight, based on 100 parts by weight of the hydrophilic compound. If the mixing ratio is within the above range, the amount of the added thiol compound, which also acts as a crosslinking agent of the hydrophilic layer, can be controlled to be optimal, the surface lubricity (including surface lubricity retention (durability) in some cases) and/or antithrombotic can be improved by means of inhibiting increase in the crosslink density of the hydrophilic layer, and excellent surface lubricity and/or antithrombotic at the time of using the medical device can be effectively maintained for a longer time. If the amount of the thiol compound added is less than 0.1 parts by weight based on the hydrophilic compound, the hydrophilic may not be sufficiently bonded (fixed) to the base material layer through the thiol compound, or a sufficiently crosslinked structure may not be formed. On the other hand, if the amount of the thiol compound added exceeds 1,000,000 parts by weight based on the hydrophilic compound, the surface lubricity (including surface lubricity retention (durability) in some cases) and/or antithrombotic at the time when the surface is wet may deteriorate due to increase in the crosslink density of the hydrophilic layer.

The solvent used for preparing the mixed solution is not particularly limited, as long as it is a solvent that can dissolve the thiol compound and the hydrophilic compound. Examples of the solvent include water, alcohols such as methanol, ethanol, isopropanol, and ethylene glycol, ketones such as acetone and methyl ethyl ketone, esters such as ethyl acetate, halides such as chloroform and methylene chloride, olefins such as butane and hexane, ethers such as tetrahydrofuran and butyl ether, aromatic solvents such as benzene and toluene, amides such as N,N-dimethylformamide (DMF), dioxane, and the like. However, the present invention is not limited to these. One kind among the above may be used alone, or two or more kinds among the above may be used concurrently.

The concentration (total amount of the added thiol compound and hydrophilic compound based on a solvent) of the compounds in the mixed solution is preferably 0.05 mg/mL to 200 mg/mL and more preferably 0.1 mg/mL to 100 mg/mL, in view of ease of obtaining the hydrophilic layer having a desired thickenss, operability (for example, ease of coating), and the like. If the concentration of the compounds in the mixed solution is within the above range, a hydrophilic layer having a desired uniform thickness can be easily obtained by one-time coating, and this is preferable in view of production efficiency. Consequently, even the thin and narrow inner surface of medical devices such as catheters, guide wires, and indwelling needles can be rapidly and easily covered with the hydrophilic layer having a desired uniform thickness. However, even though the concentration is out of the above range, if the effect of the present invention is not influenced, the mixed solution can be used excellently.

The method of coating (covering) the base material layer with the mixed solution is not particularly limited, and conventionally known methods such as a dipping method, a coating-pringing method, a spraying method, a spin coating method, and a coating method using sponge immersed with the mixed solution can be used.

When the base material layer is coated with the mixed solution (coating solution) by being dipped into the mixed solution, defoaming may be performed by reducing the internal pressure of the coating system, in a state where the base material layer has been dipped into the mixed solution. Particularly, in medical devices such as catheters, guide wires, and indwelling needles, it is possible to accelerate formation of the hydrophilic layer by causing the solution to rapidly penetrate the thin and narrow inner surface.

Moreover, when the hydrophilic layer is formed on only a portion of the base material layer, only a portion of the base material layer is dipped into the mixed solution such that the portion of the base material layer is coated with the mixed solution (coating solution), and then a reaction is caused by a heating operation and the like. In this manner, a hydrophilic layer, in which the hydrophilic compound and the thiol compound have formed a crosslinked structure, can be formed at a desired portion of the surface of the base material layer.

When it is difficult to dip only a portion of the base material layer in the mixed solution, the portion of surface, which does not require formation of the hydrophilic layer, of the base material layer is protected (covered) in advance with an appropriate member or material that can be removed (can be mounted on or detached from the base material layer), and the base material layer is dipped into the mixed solution so as to be coated with the mixed solution. Thereafter, the protective member (material) of the portion of surface, which does not require formation of the hydrophilic layer, is removed, and then a reaction is caused by a heating operation and the like. In this manner, the hydrophilic layer can be formed on a desired portion of surface of the base material layer. However, the present invention is not limited to the formation methods described above, and the hydrophilic layer can be formed by appropriately using conventionally known methods. For example, when it is difficult to dip only a portion of the base material layer in the mixed solution, other coating methods (for example, a coating method and a spraying method) may be used instead of the dipping method. In addition, due to the structure of a medical device, when both the outer and inner surfaces of the device having a cylindrical shape need to exhibit lubricity and/or antithrombotic when being wet, it is preferable to use the dipping method since both the outer and inner surfaces can be coated at a time.

The method of reacting the thiol groups of the thiol compound with the reactive functional groups of the hydrophilic compound is not particularly limited, and for example, conventionally known methods such as heating treatment, light irradiation, UV irradiation, electron beam irradiation, radiation irradiation, and plasma irradiation can be used. Particularly, the hydrophilic compound according to the present invention contains thiol groups, alkenyl groups, acryl groups, or methacryl groups as the reactive functional groups. These reactive functional groups exhibit low reactivity when they are simply in the state of a solution. However, by being subjected to treatment such as heating, UV irradiation, or plasma irradiation, the reactive functional groups can exhibit higher reactivity. Therefore, the method according to the present invention has such advantages that the reaction can be caused under relatively mild conditions, the reaction time can be shortened, and a rigid crosslinked structure can be formed since the thiol compound can firmly bind to the hydrophilic compound.

When the reaction is caused by, for example, heating treatment among the above reaction methods, conditions (reaction conditions) of the heating treatment are not particularly limited, as long as the reaction between the reactive functional groups and the thiol groups (and formation of the crosslinked structure by this reaction) and the reaction between the residual thiol groups in the crosslinked structure (in the thiol compound) and the surface of the base material layer can proceed (can be accelerated) simultaneously. The heating temperature is preferably 40°C or higher, more preferably 50°C to 150°C, and particularly preferably 60°C to 130°C. Moreover, the heating time is preferably 15 minutes or longer and shorter than 7 hours, more preferably 30 minutes to 6 hours, and particularly preferably 1 hour to 5 hours. Under the above conditions, the reaction between the reactive functional groups and the thiol groups (and formation of the crosslinked structure by this reaction) and the reaction between the residual thiol groups in the crosslinked structure (in the thiol compound) and the surface of the base material layer can proceeds (can be accelerated) rapidly.

The pressure conditions at the time of the heating treatment are not limited at all, and the heating treatment may be performed under normal pressure (atmospheric pressure), in a pressurized state, or under reduced pressure. Moreover, a reaction catalyst such as a tertiary amine compound like a trialkylamine compound or pyridine may be used by being added in an appropriate amount to the mixed solution at a proper time such that the reaction between the reactive functional groups of the hydrophilic compound and the thiol groups can be accelerated. As the heating means

(apparatus), for example, an oven, a drier, a microwave heating apparatus, and the like can be used.

Likewise, UV irradiation conditions (reaction conditions) at the time when the reaction is caused by UV irradiation are not particularly limited, as long as the reaction between the reactive functional groups and the thiol groups (and formation of the crosslinked structure by this reaction) and the reaction between the residual thiol groups in the crosslinked structure (in the thiol compound) and the surface of the base material layer can proceed (can be accelerated) simultaneously. The temperature at the time of UV irradiation is preferably 10°C to 50°C and more preferably 15°C to 35°C. Moreover, the UV irradiation time is preferably 30 seconds to 60 minutes, and more preferably 50 seconds to 30 minutes. Under the above conditions, the reaction between the reactive functional groups and the thiol groups (and formation of the crosslinked structure by this reaction) and the reaction between the residual thiol groups in the crosslinked structure (in the thiol compound) and the surface of the base material layer can proceeds (can be accelerated) rapidly. The pressure conditions at the time of the UV irradiation are not limited at all, and the UV irradiation may be performed under normal pressure (atmospheric pressure), in a pressurized state, or under reduced pressure. However, the UV irradiation is generally performed under normal pressure (atmospheric pressure).

Similarly, plasma irradiation conditions (reaction conditions) at the time when the reaction is caused by plasma irradiation are not particularly limited, as long as the reaction between the reactive functional groups and the thiol groups (and formation of the crosslinked structure by this reaction) and the reaction between the residual thiol groups in the crosslinked structure (in the thiol compound) and the surface of the base material layer can proceed (can be accelerated) simultaneously. The temperature at the time of plasma irradiation is preferably 5°C to 35°C and more preferably 15°C to 30°C. Moreover, the plasma irradiation time is preferably 10 minutes or shorter, more preferably 0.1 seconds to 5 minutes, and even more preferably 1 second to 3 minutes. Under the above conditions, the reaction between the reactive functional groups and the thiol groups (and formation of the crosslinked structure by this reaction) and the reaction between the residual thiol groups in the crosslinked structure (in the thiol compound) and the surface of the base material layer can proceeds (can be accelerated) rapidly. The pressure conditions at the time of the plasma irradiation are not limited at all, and the plasma irradiation may be performed under normal pressure (atmospheric pressure), in a pressurized state, or under reduced pressure. However, it is preferable to perform the plasma irradiation under atmospheric pressure, since plasma gas can be emitted from any angle, a vacuum apparatus is not required, the apparatus can be downsized, a space-saving system can be constituted at a low cost, and economic efficiency is excellent. In addition, if the substance to be treated, such as a guide wire, is irradiated with plasma gas emitted from a plasma emission nozzle which circles once around the substance, the whole circumference of the substance to be treated can be evenly treated with plasma without showing unevenness.

Examples of ionized gas that can be used for the plasma treatment include helium, neon, argon, krypton, carbon dioxide, carbon monoxide, water vapor, hydrogen, and the like. One kind among the above ionized gas may be used alone, or two or more kinds among the above may be used in the form of mixed gas. Moreover, the ionized gas may contain oxygen or nitrogen. The amount of oxygen or nitrogen contained in the ionized gas is not particularly limited and can be appropriately selected. The conditions of the plasma treatment, such as a current to be applied and a flow rate of gas, are not particularly limited and may be appropriately determined according to the area of the substance to be treated and the type of plasma irradiation apparatus or ionized gas to be used.

The plasma irradiation apparatus (system) that can be used for the plasma treatment is not particularly limited, and examples thereof include a plasma irradiation apparatus (system) which includes a plasma generating tube that generates plasma by exciting gas molecules put into the tube and electrodes that excite the gas molecules in the plasma generating tube, and is constituted to emit plasma from one end of the plasma generating tube. However, the plasma irradiation apparatus present invention is not limited to this constitution (system). For example, among apparatus that have come to the market, ionized gas plasma irradiation apparatus (systems) that are suitable for irradiation of catheters, guide wires, indwelling needles, and the like, particularly, plasma irradiation apparatus (systems) used under atmospheric pressure can be used. Specifically, DURADYNE (product name or trade name) as a plasma irradiation apparatus manufactured by Tri-Star Technologies., PLASMABEAM as a plasma irradiation apparatus manufactured by Diener Electronic GmbH + Co. KG, and the like can be used, but the plasma irradiation apparatus is not limited to these.

When the thiol groups of the thiol compound are reacted with the reactive functional groups of the hydrophilic compound by the aforementioned UV irradiation or ionized gas plasma irradiation, heating treatment and the like may be performed after the UV irradiation or ionized gas plasma irradiation. In this manner, the reaction between the thiol groups of the thiol compound and the reactive functional groups of the hydrophilic compound can be further accelerated. Accordingly, by the heating treatment and the like, the thiol compound can be more firmly fixed to the hydrophilic compound, and a more rigid crosslinked structure can be formed. The heating treatment conditions (reaction conditions) are not particularly limited, as long as the reaction between the reactive functional groups and the thiol groups (and formation of the crosslinked structure by this reaction) and the reaction between the residual thiol groups in the crosslinked structure (in the thiol compound) and the surface of the base material layer can proceed (can be accelerated) simultaneously. The heating temperature is preferably 40°C or higher, more preferably 50°C to 150°C, and particularly preferably 60°C to 130°C. The heating time is preferably 15 minutes or longer and shorter than 7 hours, more preferably 30 minutes to 6 hours, and particularly preferably 1 hour to 5.5 hours. Under the above conditions, the reaction between the reactive functional groups and the thiol groups (and formation of the crosslinked structure by this reaction) and the reaction between the residual thiol groups in the crosslinked structure (in the thiol compound) and the surface of the base material layer can proceeds (can be accelerated) rapidly. Likewise, the pressure conditions at the time of the heating treatment are not limited, and the heating treatment may be performed under normal pressure (atmospheric pressure), in a pressurized state, or under reduced pressure. As the heating means (apparatus), for example, an oven, a drier, a microwave heating apparatus, and the like can be used.

In addition, the surface of the base material layer may be irradiated in advance with ionized gas plasma, before the step of coating (covering) by using the mixed solution containing the thiol compound and the hydrophilic compound. The ionized gas plasma irradiation can be performed regardless of the type of the base material layer. However, it is preferable to perform ionized gas plasma irradiation on the surface of the base material layer, particularly when the base material layer is formed of a polymer material or glass, or when the surface thereof is covered with a polymer material or glass. In this manner, the surface of the base material layer is modified and activated, whereby functional groups such as carboxyl groups, hydroxyl groups, or peroxides are introduced into the surface of the base material layer. In this manner, the surface of the base material layer can be wet with the mixed solution to a higher extent and can be more uniformly coated with the hydrophilic layer. Furthermore, the aforementioned functional groups on the surface of the base material layer react with the thiol groups of the thiol compound, whereby the hydrophilic layer can be more firmly fixed to the surface of the base material layer through the thiol compound by a simple method. In addition, with the ionized gas plasma treatment, even a thin and narrow inner surface of medical device such as catheters, guide wires, and indwelling needles can be treated with plasma to a desired extent. Moreover, it is preferable to wash the surface of the base material layer in advance by an appropriate method before the surface of the base material layer is irradiated with ionized gas plasma.

The conditions of the ionized gas plasma treatment performed on the surface of the base material layer are not particularly limited. For example, the treatment may be performed under any pressure condition such as reduced pressure or atmospheric pressure. However, it is preferable to perform the ionized gas plasma treatment under atmospheric pressure, since plasma gas can be emitted from any angle, a vacuum apparatus is not required, the apparatus can be downsized, a space-saving system can be constituted at a low cost, and economic efficiency is excellent. In addition, if the substance to be treated, such as a guide wire, is irradiated with plasma gas emitted from a plasma emission nozzle which circles once around the substance, the whole circumference of the substance to be treated can be evenly treated with plasma without showing unevenness.

In the ionized gas plasma treatment, the irradiation time is 10 minutes or shorter, preferably 0.1 seconds to 5 minutes, and more preferably within a range of 1 second to 3 minutes. If the ionized gas plasma irradiation is performed for the time described above, wettability (modification or activation) of the surface of the base material layer is sufficiently improved, and a thin coat of the hydrophilic layer can be formed. Moreover, the surface of the base material layer is activated to an appropriate degree, and the reaction between residual thiol groups in the thiol compound and the surface of the base material layer can proceed (can be accelerated) rapidly.

The temperature at the time of irradiation during the ionized gas plasma treatment is not particularly limited. However, for the base material layer in which at least the surface thereof is formed of a polymer material or glass, it is preferable for the temperature to be lower than a melting point of the polymer material or glass of the surface of the base material layer and to be within a range of temperature that does not cause deformation of the base material layer. Accordingly, the temperature may be ambient temperature or may be heightened or lowered by heating or cooling. In view of economics, a temperature (5°C to 35°C) not requiring a heating apparatus or a cooling apparatus is preferable.

The conditions of the ionized gas plasma treatment, such as a current to be applied and a flow rate of gas, are not particularly limited and may be appropriately determined according to the area of the substance to be treated and the type of plasma irradiation apparatus or ionized gas to be used.

The plasma irradiation apparatus (system) that can be used for the ionized gas plasma treatment is not particularly limited, and examples thereof include a plasma irradiation apparatus (system) which includes a plasma generating tube that generates plasma by exciting gas molecules put into the tube and electrodes that excite the gas molecules in the plasma generating tube, and is constituted to emit plasma from one end of the plasma generating tube. However, the plasma irradiation apparatus is not limited to this constitution (system). For example, among apparatus that have come to the market, ionized gas plasma irradiation apparatus (systems) that are suitable for irradiation of catheters, guide wires, indwelling needles, and the like, particularly, plasma irradiation apparatus (systems) used under atmospheric pressure can be used. Specifically, DURADYNE (product name or trade name) as a plasma irradiation apparatus manufactured by Tri-Star Technologies., PLASMABEAM as a plasma irradiation apparatus manufactured by Diener Electronic GmbH + Co. KG, and the like can be used, but the plasma irradiation apparatus is not limited to these.

Examples of ionized gas that can be used for the ionized gas plasma treatment include helium, neon, argon, krypton, carbon dioxide, carbon monoxide, water vapor, hydrogen, and the like. One kind among the above ionized gas may be used alone, or two or more kinds among the above may be used in the form of mixed gas.

The ionized gas may contain oxygen. Particularly, when the base material layer is formed of a polymer material or glass, if the surface of the base material layer is irradiated with oxygen-containing ionized gas plasma, the surface of the base material layer is modified and activated, whereby a large amount of functional groups such as carboxyl groups, hydroxyl groups, and peroxides are introduced into the surface of the base material layer. As a result, the surface of the base material layer can be wet with the mixed solution to a higher extent and can be more uniformly coated with the hydrophilic layer. Furthermore, the aforementioned functional groups on the surface of the base material layer react with the residual thiol groups in the thiol compound, whereby the hydrophilic layer can be more firmly fixed to the surface of the base material layer through the thiol compound by a simple method. The amount of oxygen contained in the ionized gas is not particularly limited as long as the aforementioned effects can be obtained.

The ionized gas may contain nitrogen. Particularly, when the base material layer is formed of a polymer material or glass, if the surface of the base material layer is irradiated with nitrogen-containing ionized gas plasma, the surface of the base material layer is modified and activated, whereby functional groups such as carboxyl groups, hydroxyl groups, and peroxides are introduced into the surface of the base material layer. As a result, the surface of the base material layer can be wet with the mixed solution to a higher extent and can be more uniformly coated with the hydrophilic layer. Furthermore, the aforementioned functional groups on the surface of the base material layer react with the residual thiol groups in the thiol compound, whereby the hydrophilic layer can be more firmly fixed to the surface of the base material layer. Moreover, if the ionized gas contains nitrogen, amino groups can be introduced into the surface of the base material layer in addition to the above functional groups. Accordingly, a hydrogen bond or polar interaction is caused between the amino groups and the residual thiol groups in the thiol compound, whereby the hydrophilic layer is more stably retained on the surface of the base material layer. Therefore, the reaction between the thiol groups and functional groups such as peroxides is accelerated, whereby the hydrophilic layer can be more efficiently fixed onto the surface of the base material layer. The amount of nitrogen contained in the ionized gas is not particularly limited as long as the effects described above are obtained.

In addition, heating treatment and the like may be performed after the ionized gas plasma irradiation. In this manner, it is possible to accelerate the reaction between the surface of the base material layer and the residual thiol groups in the thiol compound. As a result, by the heating treatment and the like, the hydrophilic layer can be more firmly fixed onto the surface of the base material layer.

After the hydrophilic layer is formed as described above, the surplus hydrophilic compound, thiol compound, and the like can be washed off with an appropriate solvent such that only the crosslinked structure (formed by the reaction between the reactive functional groups and the thiol groups) formed when the hydrophilic layer is directly and firmly fixed to the base material layer remains.

The crosslinked structure which is formed as above and constitutes the hydrophilic layer absorbs water at the body temperature (30°C to 40°C) of a patient, and permanently exhibits surface lubricity and/or antithrombotic.

The medical device according to the present invention is a device used by being brought into contact with the living body fluid, blood, and the like, in the form of the any of the above embodiments. It is a device of which the surface exhibits lubricity and/or antithrombotic in aqueous liquid such as the living body fluid or physiological saline and which can improve operability or reduce the damage of mucous membranes of tissues. Specific examples thereof include guide wires, catheters, and the like that are used inside the blood vessels. In addition, the examples also include the following medical devices.

(1) Catheters orally or transnasally inserted into or indwelling in digestive organs, such as a gastric catheter, a nutrition catheter, and a feeding tube catheter
(2) Catheters orally or transnasally inserted into or indwelling in the respiratory tract or trachea, such as an oxygen catheter, an oxygen cannula, a tube or cuff of an endotracheal tube, a tube or cuff of a tracheotomy tube, and an endotracheal suction catheter
(3) Catheters inserted into or indwelling in the urethra or ureter, such as a urethral catheter, a Foley catheter, and a cather or balloon of a urethral balloon catheter
(4) Catheters inserted into or indwelling in various body cavities, organs, and tissues, such as a suction catheter, a drainage catheter, or a rectal catheter
(5) Catheters inserted into or indwelling in blood vessels, such as an indwelling needle, an IVH catheter, a thermodilution catheter, an angiographic catheter, a vasodilating catheter, a dilator, and an introducer, or a guide wire or stilet thereof
(6) An artificial trachea, an artificial bronchus, and the like
(7) Medical devices (an artificial lung, an artificial heart, an artificial kidney, and the like) for extracorporeal circulation treatment or circuits thereof

### Examples

The effects of the present invention will be described using the following examples and comparative examples. However, the technical scope of the present invention is not limited to the following examples.

### (Example 1)

A polyethylene plate (20 mm x 50 mm) was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of O-[2-(3-mercaptopropionylamino)ethyl]-O'-methyl polyethylene glycol (molecular weight of 20,000). This plate was irradiated with ionized argon gas plasma at 25°C for 25 seconds under atmospheric pressure by using a plasma irradiation apparatus (DURADYNE manufactured by Tri-Star Technologies.), followed by a reaction for 3 hours in an oven at 80°C to form a hydrophilic layer (thickness: 1 µm) on the polyethylene plate, thereby obtaining a sample (1).

The obtained sample (1) was evaluated in terms of antithrombotic in the following manner. That is, a solution containing human blood: 3.8wt% aqueous solution of sodium citrate (= about 10:1 (volume ratio)) was subjected to centrifugation for 5 minutes at 1,200 rpm, and the supernatant platelet-rich blood plasma (PRP) was collected. Moreover, the residue was subjected to centrifugation for 10 minutes at 3,000 rpm, and the supernatant platelet-poor blood plasma (PPP) was collected. The PRP and PPP were diluted to yield a platelet count of 1 × 10⁵/mL, thereby preparing a diluted solution. The diluted solution was dropped in an amount of 200 µL onto the surface of the sample (1), and the sample was left at room temperature for 30 minutes. Thereafter, the sample (1) was washed with a PBS solution and then fixed in a 1 wt% glutaraldehyde/PBS solution for 24 hours. The sample having undergone fixation was sufficiently washed with distilled water, and then observed under a scanning electron microscope in three visual fields within a range of 2.2 mm x 3.3 mm. In this manner, the number of platelets adhered to the sample was counted, and the averages of the platelet counts in three visual fields were compared to each other.

The results are shown in Table 1.

### (Comparative example 1)

A sample (2) was prepared in the same manner as in Example 1, except that the polyethylene plate (20 mm x 50 mm) was dipped into 25 mL of a mixed solution of acetone-methylene chloride (96:4 (volume ratio)) containing only 250 mg of tris-[(3-mercaptopropionyloxy)-ethyl]-isocyanurate (TEMPIC) (manufactured by SC Organic Chemical Co., Ltd.; having three thiol groups in a single molecule) in Example 1.

The obtained sample (2) was evaluated in terms of antithrombotic in the same manner as in Example 1. The result is shown in Table 1.

### (Comparative example 2)

A sample (3) was prepared in the same manner as in Example 1, except that the polyethylene plate (20 mm x 50 mm) was dipped into 25 mL of a mixed solution of acetone-methylene chloride (96:4 (volume ratio)) containing nothing in Example 1.

The obtained sample (3) was evaluated in terms of antithrombotic in the same manner as in Example 1. The result is shown in Table 1.

**[Table 1]**

| | Example 1 Sample (1) | Comparative example 1 Sample (2) | Comparative example 2 Sample (3) |
|---|---|---|---|
| Visual field 1 | 0 | 603 | 592 |
| Visual field 2 | 0 | 632 | 652 |
| Visual field 3 | 1 | 560 | 560 |
| Average | 0.3 | 598 | 601 |

Table 1 shows that the number of platelets adhered to the sample (1) of the present invention that has a hydrophilic layer formed of a thiol compound and a hydrophilic compound on the surface is smaller than that of the platelets adhered to the sample (3) that has only a polyethylene base material layer. The table also shows that adhesion of platelet to the sample (2) that has a layer formed only of a thiol compound on the surface cannot be suppressed, and the sample (2) exhibits practically the same result as the sample (3).

### (Example 2)

A plate (20 mm x 50 mm) made of SUS304 was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of O-[2-(3-mercaptopropionylamino)ethyl]-O'-methyl polyethylene glycol (molecular weight of 20,000), followed by a reaction for three hours in an oven at 120°C to form a hydrophilic layer (thickness: 1 µm) on the SUS plate, thereby obtaining a sample (4).

The obtained sample (4) was evaluated in terms of antithrombotic in the same manner as in Example 1. The result is shown in Table 2.

### (Comparative example 3)

A sample (5) was prepared in the same manner as in Example 2, except that the plate (20 mm x 50 mm) made of SUS 304 was dipped into a 25 mL of a mixed solution of acetone-methylene chloride (96:4 (volume ratio)) containing only 250 mg of tris-[(3-mercaptopropionyloxy)-ethyl]-isocyanurate (TEMPIC) (manufactured by SC Organic Chemical Co., Ltd.; having three thiol groups in a single molecule) in Example 2.

The obtained sample (5) was evaluated in terms of antithrombotic in the same manner as in Example 1. The result is shown in Table 2.

### (Comparative example 4)

A sample (6) was prepared in the same manner as in Example 2, except that the plate (20 mm x 50 mm) made of SUS304 was dipped into 25 mL of a mixed solution of acetone-methylene chloride (96:4 (volume ratio)) containing nothing in Example 2.

The obtained sample (6) was evaluated in terms of antithrombotic in the same manner as in Example 1. The result is shown in Table 2.

### (Example 3)

A polyethylene plate (20 mm x 50 mm) was dipped into 25 mL of a mixed solution of acetone-methylene chloride (96:4 (volume ratio)) containing 250 mg of dipentaerythritol hexakis(3-mercaptopropionate) (manufactured by SC Organic Chemical Co., Ltd.; having six thiol groups in a single molecule) and 250 mg of O-[2-(3-mercaptopropionylamino)ethyl]-O'-methyl polyethylene glycol (molecular weight of 20,000). This plate was irradiated with ionized argon gas plasma at 25°C for 25 seconds under atmospheric pressure by using a plasma irradiation apparatus (DURADYNE manufactured by Tri-Star Technologies.), followed by a reaction for 3 hours in an oven at 80°C to form a hydrophilic layer (thickness: 1 µm) on the polyethylene plate, thereby obtaining a sample (7).

The obtained sample (7) was evaluated in terms of antithrombotic in the same manner as in Example 1. The result is shown in Table 2.

### (Comparative example 5)

A sample (8) was prepared in the same manner as in Example 3, except that the polyethylene plate (20 mm x 50 mm) was dipped into 25 mL of a mixed solution of acetone-methylene chloride (96:4 (volume ratio)) containing only 250 mg of dipentaerythritol hexakis(3-mercaptopropionate) (manufactured by SC Organic Chemical Co., Ltd.; having six thiol groups in a single molecule) in Example 3.

The obtained sample (8) was evaluated in terms of antithrombotic in the same manner as in Example 1. The result is shown in Table 2.

### (Comparative example 6)

A sample (9) was prepared in the same manner as in Example 3, except that the polyethylene plate (20 mm x 50 mm) was dipped into 25 mL of a mixed solution of acetone-methylene chloride (96:4 (volume ratio)) containing nothing in Example 3.

The obtained sample (9) was evaluated in terms of antithrombotic in the same manner as in Example 1. The result is shown in Table 2.

### (Example 4)

A polyethylene plate (20 mm x 50 mm) was dipped into 25 mL of a mixed solution of acetone-methylene chloride (96:4 (volume ratio)) containing 250 mg of tetraethylene glycol bis(3-mercaptopropionate) (manufactured by SC Organic Chemical Co., Ltd.; having two thiol groups in a single molecule) and 250 mg of O-[2-(3-mercaptopropionylamino)ethyl]-O'-methyl polyethylene glycol (molecular weight of 20,000). This plate was irradiated with ionized argon gas plasma at 25°C for 25 seconds under atmospheric pressure by using a plasma irradiation apparatus (DURADYNE manufactured by Tri-Star Technologies.), followed by a reaction for 3 hours in an oven at 80°C to form a hydrophilic layer (thickness: 1 µm) on the polyethylene plate, thereby obtaining a sample (10).

The obtained sample (10) was evaluated in terms of antithrombotic in the same manner as in Example 1. The result is shown in Table 2.

### (Comparative example 7)

A sample (11) was prepared in the same manner as in Example 4, except that the polyethylene plate (20 mm x 50 mm) was dipped into 25 mL of a mixed solution of acetone-methylene chloride (96:4 (volume ratio)) containing only 250 mg of tetraethylene glycol bis(3-mercaptopropionate) (manufactured by SC Organic Chemical Co., Ltd.; having two thiol groups in a single molecule) in Example 4.

The obtained sample (11) was evaluated in terms of antithrombotic in the same manner as in Example 1. The result is shown in Table 2.

### (Comparative example 8)

A sample (12) was prepared in the same manner as in Example 4, except that the polyethylene plate (20 mm x 50 mm) was dipped into 25 mL of a mixed solution of acetone-methylene chloride (96:4 (volume ratio)) containing nothing in Example 4.

The obtained sample (12) was evaluated in terms of antithrombotic in the same manner as in Example 1. The result is shown in Table 2.

**[Table 2]**

| | Platelet count (average) |
|---|---|
| Example 2 Sample (4) | 0.3 |
| Comparative example 3 Sample (5) | 597 |
| Comparative example 4 Sample (6) | 605 |
| Example 3 Sample (7) | 0.3 |
| Comparative example 5 Sample (8) | 607 |
| Comparative example 6 Sample (9) | 595 |
| Example 4 Sample (10) | 0.3 |
| Comparative example 7 Sample (11) | 596 |
| Comparative example 8 Sample (12) | 593 |

Table 2 shows that the number of platelets adhered to each of the samples (4), (7), and (10) according to the present invention that has the hydrophilic layer formed of a thiol compound and a hydrophilic compound on the surface can be suppressed to be smaller than that of platelets adhered to the samples (6), (9), and (12) that has only a SUS base material layer or only a polyethylene base material layer and the samples (5), (8), and (11) that has a layer formed only of a thiol compound on the surface.

### (Example 5)

A polyethylene plate (20 mm x 50 mm) was dipped into 25 mL of an acetone solution containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of 3-mercapto-1-propanol. This plate was irradiated with ionized argon gas plasma at 25°C for 25 seconds under atmospheric pressure by using a plasma irradiation apparatus (DURADYNE manufactured by Tri-Star Technologies.), followed by a reaction for 3 hours in an oven at 80°C to form a hydrophilic layer (thickness: 1 µm) on the polyethylene plate, thereby obtaining a sample (13).

The obtained sample (13) was evaluated in terms of antithrombotic in the same manner as in Example 1. The result is shown in Table 3.

### (Example 6)

A polyethylene plate (20 mm x 50 mm) was dipped into 25 mL of an acetone solution containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of 3-mercaptopropionic acid. This plate was irradiated with ionized argon gas plasma at 25°C for 25 seconds under atmospheric pressure by using a plasma irradiation apparatus (DURADYNE manufactured by Tri-Star Technologies.), followed by a reaction for 3 hours in an oven at 80°C to form a hydrophilic layer (thickness: 1 µm) on the polyethylene plate, thereby obtaining a sample (14).

The obtained sample (14) was evaluated in terms of antithrombotic in the same manner as in Example 1. The result is shown in Table 3.

### (Example 7)

A polyethylene plate (20 mm x 50 mm) was dipped into 25 mL of an acetone solution containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of α-thioglycerol. This plate was irradiated with ionized argon gas plasma at 25°C for 25 seconds under atmospheric pressure by using a plasma irradiation apparatus (DURADYNE manufactured by Tri-Star Technologies.), followed by a reaction for 3 hours in an oven at 80°C to form a hydrophilic layer (thickness: 1 µm) on the polyethylene plate, thereby obtaining a sample (15).

The obtained sample (15) was evaluated in terms of antithrombotic in the same manner as in Example 1. The result is shown in Table 3.

### (Example 8)

A polyethylene plate (20 mm x 50 mm) was dipped into 25 mL of a mixed solution of acetone-water (95:5 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of sodium 2-mercaptoethanesulfonate. This plate was irradiated with ionized argon gas plasma at 25°C for 25 seconds under atmospheric pressure by using a plasma irradiation apparatus (DURADYNE manufactured by Tri-Star Technologies.), followed by a reaction for 3 hours in an oven at 80°C to form a hydrophilic layer (thickness: 1 µm) on the polyethylene plate, thereby obtaining a sample (16).

The obtained sample (16) was evaluated in terms of antithrombotic in the same manner as in Example 1. The result is shown in Table 3.

### (Example 9)

A polyethylene plate (20 mm x 50 mm) was dipped into 25 mL of an acetone solution containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of N,N-dimethylacrylamide. This plate was irradiated with UV for 60 seconds at room temperature (25°C), followed by a reaction for three hours in an oven at 80°C to form a hydrophilic layer (thickness: 1 µm) in the polyethylene plate, thereby obtaining a sample (17).

The obtained sample (17) was evaluated in terms of antithrombotic in the same manner as in Example 1. The result is shown in Table 3.

### (Example 10)

A polyethylene plate (20 mm x 50 mm) was dipped into 25 mL of an acetone solution containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of 2-hydroxyethylmethacryalte. This plate was irradiated with UV for 60 seconds at room temperature (25°C), followed by a reaction for three hours in an oven at 80°C to form a hydrophilic layer (thickness: 1 µm) in the polyethylene plate, thereby obtaining a sample (18).

The obtained sample (18) was evaluated in terms of antithrombotic in the same manner as in Example 1. The result is shown in Table 3.

### (Comparative example 9)

A polyethylene plate (20 mm x 50 mm) was dipped into 25 mL of an acetone solution containing only 250 mg of tris-[(3-mercaptopropionyloxy)-ethyl]-isocyanurate (TEMPIC) (manufactured by SC Organic Chemical Co., Ltd.; having three thiol groups in a single molecule). This plate was irradiated with ionized argon gas plasma at 25°C for 25 seconds under atmospheric pressure by using a plasma irradiation apparatus (DURADYNE manufactured by Tri-Star Technologies.), followed by a reaction for 3 hours in an oven at 80°C to form a hydrophilic layer (thickness: 1 µm) on the polyethylene plate, thereby obtaining a sample (19).

The obtained sample (19) was evaluated in terms of antithrombotic in the same manner as in Example 1. The result is shown in Table 3.

### (Comparative example 10)

A sample (20) was prepared in the same manner as in Example 5, except that the polyethylenen pate (20 mm x 50 mm) was dipped into an acetone solution.

The obtained sample (20) was evaluated in terms of antithrombotic in the same manner as in Example 1. The result is shown in Table 3.

**[Table 3]**

| | Platelet count (average) |
|---|---|
| Example 5 Sample (13) | 0.3 |
| Example 6 Sample (14) | 0.3 |
| Example 7 Sample (15) | 0.3 |
| Example 8 Sample (16) | 0.3 |
| Example 9 Sample (17) | 0.3 |
| Example 10 Sample (18) | 0.3 |
| Comparative example 9 Sample (19) | 595 |
| Comparative example 10 Sample (20) | 604 |

Table 3 shows that the number of platelets adhered to each of samples (13) to (18) according to the present invention that has a hydrophilic layer formed of a thiol compound and a hydrophilic compound on the surface can be suppressed to be lower than that of platelets adhered to the sample (20) that has only a polyethylene base material layer and the sample (19) that has a layer formed only of a thiol compound on the surface.

### (Example 11)

A wire (φ 0.7 mm, length of 150 mm) made of SUS 304 was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of O-[2-(3-mercaptopropionylamino)ethyl]-O'-methyl polyethylene glycol (molecular weight of 20,000), followed by a reaction for 3 hours in an oven at 120°C to form a hydrophilic layer (thickness: 1 µm) on the wire made of SUS304, thereby obtaining a sample (21).

The obtained sample (21) was evaluated in terms of antithrombotic in the same manners as in Example 1. The result is shown in Table 4.

Moreover, the obtained sample (21) was evaluated in terms of surface lubricity and surface lubricity retention in the following manner by using a friction measuring apparatus (Handy Tribomaster TL201 manufactured by Trinity Lab INC.) 20 shown in Fig. 2. That is, the sample (21)13 was attached to the inside of the petri dish 12 and dipped into the water 11 having a depth by which the whole sample (21)13 is immersed. The petri dish was loaded on the friction measuring apparatus 20 shown in Fig. 2, a load was applied to the terminal 14 by the weight 15, and the sample (21)13 was caused to repeatedly slide 50 times under the following conditions to measure the value of slide resistance at that time.

### <Conditions for measuring value of slide resistance>

Speed: 16.7 mm/min
Load: 50 g
Movement distance: 25 mm
Terminal: columnar terminal made of polyethylene (φ 20 mm, R 1 mm)
Sampling speed: 10 m/sec

The results are shown in Tables 3 and 4. The value of slide resistance shown in Table 4 is an index of surface lubricity. The lower the value is, the better the surface lubricity is. In the table, "○" indicates that the value of slid resistance measured when the sample has slid once is 40 gf or less (the surface lubricity is excellent), and "×" indicates that the value of slide resistance measured when the sample has slid once is greater than 40 gf (the surface lubricity is poor). In addition, the slide retention show in Table 4 is an index of surface lubricity retention. The lower the value is, the better the surface lubricity retention. In the same table, "○" indicates that the value of slide resistance measured when the sample has slid 30 times is 40 gf or lower, and the change in the value of slide resistance measured from when the sample has slid once to when the sample has slid 30 times is within 20% (surface lubricity retention is excellent). "×" indicates that the value of slide resistance measured when the sample has slid 30 times is greater than 40 gf, or the change in the value of slide resistance measured from when the sample has slid once to when the sample has slid 30 times is greater than 20% (surface lubricity retention is poor).

In Table 4, "TEMPIC" indicates tris-[(3-mercaptopropionyloxy)-ethyl]-isocyanurate; "DPMP" indicates dipentaerythritol hexakis(3-mercaptopropionate); "EMGP-4" indicates tetraethylene glycol bis(3-mercaptopropionate); "PEG-SH" indicates 0-[2-(3-meraptopropionylamino)ethyl]-O'-methylpolyethylene glycol; "PEG-SH2" indicates mercaptopolyethylene glycol; and "PEG-MA" indicates polyethylene glycol methyl ether methacrylate respectively.

### (Comparative example 11)

A sample (22) was prepared in the same manner as in Example 11, except that the wire (φ 0.7 mm, length of 150 mm) made of SUS304 was dipped into 25 mL of a mixed solution of acetone-methylene chloride (96:4 (volume ratio)) containing only 250 mg of O-[2-(3-meraptopropionylamino)ethyl]-O'-methylpolyethylene glycol (molecular weight of 20,000) in Example 11.

The obtained sample (22) was evaluated in terms of antithrombotic in the same manner as in Example 1. The result is shown in Table 4. Moreover, the obtained sample (22) was evaluated in terms of surface lubricity and surface lubricity retention in the same manner as in Example 11. The results are shown in Fig. 3 and Table 4.

### (Comparative example 12)

A sample (23) was prepared in the same manner as in Example 11, except that the wire (φ 0.7 mm, length of 150 mm) made of SUS304 was dipped into 25 mL of a mixed solution of acetone-methylene chloride (96:4 (volume ratio)) containing only 250 mg of tris-[(3-mercaptopropionyloxy)-ethyl]-isocyanurate (TEMPIC) (manufactured by SC Organic Chemical Co., Ltd.; having three thiol groups in a single molecule) in Example 11.

The obtained sample (23) was evaluated in terms of antithrombotic in the same manner as in Example 1. The result is shown in Table 4. Moreover, the obtained sample (23) was evaluated in terms of surface lubricity and surface lubricity retention in the same manner as in Example 11. The results are shown in Fig. 3 and Table 4.

### (Comparative example 13)

A sample (24) was prepared in the same manner as in Example 11, except that the wire (φ 0.7 mm, length of 150 mm) made of SUS304 was dipped into 25 mL of a mixed solution of acetone-methylene chloride (96:4 (volume ratio)) containing nothing in Example 11.

The obtained sample (24) was measured in terms of antithrombotic in the same manner as in Example 1. The result is shown in Table 4. Moreover, the obtained sample (24) was evaluated in terms of surface lubricity and surface lubricity retention in the same manner as in Example 11. The results are shown in Fig. 3 and Table 4.

As shown in Fig. 3 and Table 4, in the sample (21) obtained in Example 11, the value of slide resistance was significantly lowered, the value of slide resistance could be kept low, and durability of the hydrophilic layer was excellent, compared to the sample (24) obtained in Comparative example 13 including only a SUS base material (wire made of SUS304). Furthermore, in the sample (22) obtained in Comparative example 11 having a layer formed only of a hydrophilic compound, the initial value of slide resistance was low. However, from when the sample had slid 7 times, the value of slide resistance increased, and this showed that the value of slide resistance cannot be kept low, and the durability is poor. In the sample (23) obtained in Comparative example 12 having a layer formed only of a thiol compound, the value of slide resistance was increased significantly, compared to the sample (21) obtained in Example 11.

### (Example 12)

A wire (φ 0.7 mm, length of 150 mm) made of Ni-Ti was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of O-[2-(3-mercaptopropionylamino)ethyl]-O'-methyl polyethylene glycol (molecular weight of 20,000), followed by a reaction for 3 hours in an oven at 120°C to form a hydrophilic layer (thickness: 1 µm) on the wire made of Ni-Ti, thereby obtaining a sample (25).

The obtained sample (25) was evaluated in terms of antithrombotic in the same manner as in Example 1. The result is shown in Table 4. Moreover, the obtained sample (25) was evaluated in terms of surface lubricity and surface lubricity retention in the same manner as in Example 11. The results are shown in Table 4.

### (Comparative example 14)

A sample (26) was prepared in the same manner as in Example 12, except that the wire (φ 0.7 mm, length of 150 mm) made of Ni-Ti was dipped into 25 mL of a mixed solution of acetone-methylene chloride (96:4 (volume ratio)) containing only 250 mg of O-[2-(3-mercaptopropionylamino)ethyl]-O'-methyl polyethylene glycol (molecular weight of 20,000) in Example 12.

The obtained sample (26) was evaluated in terms of antithrombotic in the same manner as in Example 1. The result is shown in Table 4. Moreover, the obtained sample (26) was evaluated in terms of surface lubricity and surface lubricity retention in the same manner as in Example 11. The results are shown in Table 4.

### (Comparative example 15)

A sample (27) was prepared in the same manner as in Example 12, except that the wire (φ 0.7 mm, length of 150 mm) made of Ni-Ti was dipped into 25 mL of a mixed solution of acetone-methylene chloride (96:4 (volume ratio)) containing only 250 mg of tris-[(3-mercaptopropionyloxy)-ethyl]-isocyanurate (TEMPIC) (manufactured by SC Organic Chemical Co., Ltd.; having three thiol groups in a single molecule) in Example 12.

The obtained sample (27) was evaluated in terms of antithrombotic in the same manner as in Example 1. The result is shown in Table 4. Moreover, the obtained sample (27) was evaluated in terms of surface lubricity and surface lubricity retention in the same manner as in Example 11. The results are shown in Table 4.

### (Comparative example 16)

A sample (28) was prepared in the same manner as in Example 12, except that the wire (φ 0.7 mm, length of 150 mm) made of Ni-Ti was dipped into 25 mL of a mixed solution of acetone-methylene chloride (96:4 (volume ratio)) containing nothing in Example 12.

The obtained sample (28) was evaluated in terms of antithrombotic in the same manner as in Example 1. The result is shown in Table 4. Moreover, the obtained sample (28) was evaluated in terms of surface lubricity and surface lubricity retention in the same manner as in Example 11. The results are shown in Table 4.

### (Example 13)

A hollow tube (inner diameter of 0.68 mm, outer diameter of 0.87 mm) made of polyethylene (linear low-density polyethylene; Niporon-Z (registered trademark) ZF-260 manufactured by Tosoh Corporation) was subjected to ultrasonic cleaning in acetone and then treated with ionized argon gas plasma at 25°C for 25 seconds under atmospheric pressure by using a plasma irradiation apparatus (DURADYNE manufactured by Tri-Star Technologies.).

The polyethylene tube having undergone the above plasma treatment was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of O-[2-(3-mercaptopropionylamino)ethyl]-O'-methyl polyethylene glycol (molecular weight of 20,000), and then dried at 25°C. Subsequently, the polyethylene tube was irradiated again with ionized argon gas plasma at 25°C for 25 seconds under atmospheric pressure by using a plasma irradiation apparatus (DURADYNE manufactured by Tri-Star Technologies.). Thereafter, the tube was heated for 5 hours in an oven at 80°C to fix the hydrophilic layer (thickness: 1 µm) onto the surface of the polyethylene tube. The polyethylene tube onto which the hydrophilic layer had been fixed was subjected to ultrasonic cleaning in THF to remove TEMPIC and O-[2-(3-mercaptopropionylamino)ethyl]-O'-methyl polyethylene glycol that had not been fixed onto the surface, thereby preparing a sample (29). Then a stainless steel bar (φ 0.8 mm) was passed through the sample (29) and the resultant was attached to the inside of the petri dish 12 shown in Fig. 2. The surface lubricity and surface lubricity retention thereof were evaluated in the same manner as in Example 1. The results are shown in Table 4.

The obtained sample (29) was evaluated in terms of antithrombotic in the same manner as in Example 1. The result is shown in Table 4.

### (Comparative example 17)

A polyethylene tube was subjected to plasma treatment in the same manner as in Example 13. Thereafter, a sample (30) was prepared in the same manner as in Example 13, except that the polyethylene tube having undergone the plasma treatment was dipped into 25 mL of a mixed solution of acetone-methylene chloride (96:4 (volume ratio)) containing only 250 mg of O-[2-(3-mercaptopropionylamino)ethyl]-O'-methyl polyethylene glycol (molecular weight of 20,000).

The obtained sample (30) was evaluated in terms of antithrombotic in the same manner as in Example 1. The result is shown in Table 4. Moreover, the obtained sample (30) was evaluated in terms of surface lubricity and surface lubricity retention in the same manner as in Example 11. The results are shown in Table 4.

### (Comparative example 18)

A polyethylene tube was subjected to plasma treatment in the same manner as in Example 13. Thereafter, a sample (31) was prepared in the same manner as in Example 13, except that the polyethylene tube having undergone the plasma treatment was dipped into 25 mL of a mixed solution of acetone-methylene chloride (96:4 (volume ratio)) containing only 250 mg of tris-[(3-mercaptopropionyloxy)-ethyl]-isocyanurate (TEMPIC) (manufactured by SC Organic Chemical Co., Ltd.; having three thiol groups in a single molecule).

The obtained sample (31) was evaluated in terms of antithrombotic in the same manner as in Example 1. The result is shown in Table 4. Moreover, the obtained sample (31) was evaluated in terms of surface lubricity and surface lubricity retention in the same manner as in Example 11. The results are shown in Table 4.

### (Comparative example 19)

A polyethylene tube was subjected to plasma treatment in the same manner as in Example 13. Thereafter, a sample (32) was prepared in the same manner as in Example 13, except that the polyethylene tube having undergone the plasma treatment was dipped into 25 mL of a mixed solution of acetone-methylene chloride (96:4 (volume ratio)) containing nothing.

The obtained sample (32) was evaluated in terms of antithrombotic in the same manner as in Example 1. The result is shown in Table 4. Moreover, the obtained sample (32) was evaluated in terms of surface lubricity and surface lubricity retention in the same manner as in Example 11. The results are shown in Table 4.

### (Example 14)

A piece of slide glass (micro-slide glass White cut edge No. 1 manufactured by Matsunami Glass Ind., Ltd., 76 mm x 26 mm, thickness of 1 mm) was subjected to ultrasonic cleaning in acetone and then treated with ionized argon gas plasma at 25°C for 25 seconds under atmospheric pressure by using a plasma irradiation apparatus (DURADYNE manufactured by Tri-Star Technologies.).

The slide glass having undergone the above plasma treatment was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of O-[2-(3-mercaptopropionylamino)ethyl]-O'-methyl polyethylene glycol (molecular weight of 20,000), and then dried at 25°C. Subsequently, the slide glass was irradiated again with ionized argon gas plasma at 25°C for 25 seconds under atmospheric pressure by using a plasma irradiation apparatus (DURADYNE manufactured by Tri-Star Technologies.). Thereafter, the slide glass was heated for 5 hours in an oven at 80°C to fix the hydrophilic layer (thickness: 1 µm) onto the surface of the slide glass. The slide glass onto which the hydrophilic layer had been fixed was subjected to ultrasonic cleaning in THF to remove TEMPIC and O-[2-(3-mercaptopropionylamino)ethyl]-O'-methyl polyethylene glycol that had not been fixed onto the surface, thereby preparing a sample (33). Subsequently, the sample (33) was attached to the inside of the petri dish 12. The surface lubricity and surface lubricity retention thereof were evaluated in the same manner as in Example 1. The results are shown in Table 4.

Moreover, the obtained sample (33) was evaluated in terms of antithrombotic in the same manner as in Example 1. The result is shown in Table 4.

### (Comparative example 20)

A piece of slide glass was subjected to plasma treatment in the same manner as in Example 14. Thereafter, a sample (34) was prepared in the same manner as in Example 14, except that the slide glass having undergone the plasma treatment was dipped into 25 mL of a mixed solution of acetone-methylene chloride (96:4 (volume ratio)) containing only 250 mg of O-[2-(3-mercaptopropionylamino)ethyl]-O'-methyl polyethylene glycol (molecular weight of 20,000).

The obtained sample (34) was evaluated in terms of antithrombotic in the same manner as in Example 1. The result is shown in Table 4. Moreover, the obtained sample (34) was evaluated in terms of surface lubricity and surface lubricity retention in the same manner as in Example 11. The results are shown in Table 4.

### (Comparative example 21)

A piece of slide glass was subjected to plasma treatment in the same manner as in Example 14. Thereafter, a sample (35) was prepared in the same manner as in Example 14, except that the slide glass having undergone the plasma treatment was dipped into 25 mL of a mixed solution of acetone-methylene chloride (96:4 (volume ratio)) containing only 250 mg of tris-[(3-mercaptopropionyloxy)-ethyl]-isocyanurate (TEMPIC) (manufactured by SC Organic Chemical Co., Ltd.; having three thiol groups in a single molecule).

The obtained sample (35) was evaluated in terms of antithrombotic in the same manner as in Example 1. The result is shown in Table 4. Moreover, the obtained sample (35) was evaluated in terms of surface lubricity and surface lubricity retention in the same manner as in Example 11. The results are shown in Table 4.

### (Comparative example 22)

A piece of slide glass was subjected to plasma treatment in the same manner as in Example 14. Thereafter, a sample (36) was prepared in the same manner as in Example 14, except that the slide glass having undergone the plasma treatment was dipped into 25 mL of a mixed solution of acetone-methylene chloride (96:4 (volume ratio)) containing nothing.

The obtained sample (36) was evaluated in terms of antithrombotic in the same manner as in Example 1. The result is shown in Table 4. Moreover, the obtained sample (36) was evaluated in terms of surface lubricity and surface lubricity retention in the same manner as in Example 11. The results are shown in Table 4.

### (Example 15)

A wire (φ 0.7 mm, length of 150 mm) made of SUS304 was dipped into 25 mL of a mixed solution of acetone-methylene chloride (96:4 (volume ratio)) containing 250 mg of dipentaerythritol hexakis(3-mercaptopropionate) (manufactured by SC Organic Chemical Co., Ltd.; having six thiol groups in a single molecule) and 250 mg of O-[2-(3-mercaptopropionylamino)ethyl]-O'-methyl polyethylene glycol (molecular weight of 20,000), followed by a reaction for 3 hours in an oven at 120°C to form a hydrophilic layer (thickness: 1 µm) on the wire made of SUS304, thereby obtaining a sample (37).

The obtained sample (37) was evaluated in terms of antithrombotic in the same manner as in Example 1. The result is shown in Table 4. Moreover, the obtained sample (37) was evaluated in terms of surface lubricity and surface lubricity retention in the same manner as in Example 11. The results are shown in Table 4.

### (Comparative example 23)

A sample (38) was prepared in the same manner as in Example 15, except that the wire (φ 0.7 mm, length of 150 mm) made of SUS304 was dipped into 25 mL of a mixed solution of acetone-methylene chloride (96:4 (volume ratio)) containing only 250 mg of dipentaerythritol hexakis(3-mercaptopropionate) in Example 15.

The obtained sample (38) was evaluated in terms of antithrombotic in the same manner as in Example 1. The result is shown in Table 4. Moreover, the obtained sample (38) was evaluated in terms of surface lubricity and surface lubricity retention in the same manner as in Example 11. The results are shown in Table 4.

### (Example 16)

A wire (φ 0.7 mm, length of 150 mm) made of SUS304 was dipped into 25 mL of a mixed solution of acetone-methylene chloride (96:4 (volume ratio)) containing 250 mg of tetraethylene glycol bis(3-mercaptopropionate) (manufactured by SC Organic Chemical Co., Ltd.; having two thiol groups in a single molecule) and 250 mg of O-[2-(3-mercaptopropionylamino)ethyl]-O'-methyl polyethylene glycol (molecular weight of 20,000),followed by a reaction for 3 hours in an oven at 120°C to form a hydrophilic layer (thickness: 1 µm) on the wire made of SUS304, thereby obtaining a sample (39).

The obtained sample (39) was evaluated in terms of antithrombotic in the same manner as in Example 1. The result is shown in Table 4. Moreover, the obtained sample (39) was evaluated in terms of surface lubricity and surface lubricity retention in the same manner as in Example 11. The results are shown in Table 4.

### (Comparative example 24)

A sample (40) was prepared in the same manner as in Example 16, except that the wire (φ 0.7 mm, length of 150 mm) made of SUS304 was dipped into 25 mL of a mixed solution of acetone-methylene chloride (96:4 (volume ratio)) containing only 250 mg of tetraethylene glycol bis(3-mercaptopropionate) in Example 16.

The obtained sample (40) was evaluated in terms of antithrombotic in the same manner as in Example 1. The result is shown in Table 4. Moreover, the obtained sample (40) was evaluated in terms of surface lubricity and surface lubricity retention in the same manner as in Example 11. The results are shown in Table 4.

### (Example 17)

A wire (φ 0.7 mm, length of 150 mm) made of SUS304 was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of mercaptopolyethylene glycol (manufactured by NOF CORPORATION, SUNBRIGHT (registered trademark) SH Series Thiol PEGs, molecular weight of 40,000), followed by a reaction for 3 hours in an oven at 120°C to form a hydrophilic layer (thickness: 1 µm) on the wire made of SUS 304, thereby forming a sample (41).

The obtained sample (41) was evaluated in terms of antithrombotic in the same manner as in Example 1. The result is shown in Table 4. Moreover, the obtained sample (41) was evaluated in terms of surface lubricity and surface lubricity retention in the same manner as in Example 11. The results are shown in Table 4.

### (Comparative example 25)

A sample (42) was prepared in the same manner as in Example 17, except that the wire (φ 0.7 mm, length of 150 mm) made of SUS304 was dipped into 25 mL of a mixed solution of acetone-methylene chloride (96:4 (volume ratio)) containing only 250 mg of mercaptopolyethylene glycol (manufactured by NOF CORPORATION, SUNBRIGHT (registered trademark) SH Series Thiol PEGs, molecular weight of 40,000) in Example 17.

The obtained sample (42) was evaluated in terms of antithrombotic in the same manner as in Example 1. The result is shown in Table 4. Moreover, the obtained sample (42) was evaluated in terms of surface lubricity and surface lubricity retention in the same manner as in Example 11. The results are shown in Table 4.

### (Example 18)

A hallow tube (inner diameter of 0.68 mm, outer diameter of 0.87 mm) made of polyethylene (linear low-density polyethylene; Niporon-Z (registered trademark) ZF-260 manufactured by Tosoh Corporation) was subjected to ultrasonic cleaning in acetone and then treated with ionized argon gas plasma at 25°C for 25 seconds under atmospheric pressure by using a plasma irradiation apparatus (DURADYNE manufactured by Tri-Star Technologies.).

The polyethylene tube having undergone the plasma treatment was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg of O-[2-(3-mercaptopropionylamino)ethyl]-O'-methyl polyethylene glycol (molecular weight of 20,000), dried at 25°C, and then irradiated with UV at room temperature (25°C) for 10 minutes. Thereafter, the polyethylene tube was heated for 5 hours in an oven at 80°C to fix a hydrophilic layer (thickness: 1 µm) onto the surface of the polyethylene tube. The polyethylene tube onto which the hydrophilic layer had been fixed was then subjected to ultrasonic cleaning in THF to remove TEMPIC and O-[2-(3-mercaptopropionylamino)ethyl]-O'-methyl polyethylene glycol that had not been fixed onto the surface, thereby preparing a sample (43). Subsequently, a stainless steel bar (φ 0.8 mm) was passed through the sample (43), and the resultant was attached to the inside of the petri dish 12 shown in Fig. 2. The surface lubricity and surface lubricity retention thereof were evaluated in the same manner as in Example 1. The results are shown in Table 4.

The obtained sample (43) was evaluated in terms of antithrombotic in the same manner as in Example 1. The result is shown in Table 4.

### (Comparative example 26)

A polyethylene tube was subjected to plasma treatment in the same manner as in Example 18. Thereafter, a sample (44) was prepared in the same manner as in Example 18, except that the polyethylene tube having undergone the plasma treatment was dipped into 25 mL of a mixed solution of acetone-methylene chloride (96:4 (volume ratio)) containing only 250 mg of O-[2-(3-mercaptopropionylamino)ethyl]-O'-methyl polyethylene glycol (molecular weight of 20,000).

The obtained sample (44) was evaluated in terms of antithrombotic in the same manner as in Example 1. The result is shown in Table 4. Moreover, the obtained sample (44) was evaluated in terms of surface lubricity and surface lubricity retention in the same manner as in Example 11. The results are shown in Table 4.

### (Comparative example 27)

A polyethylene tube was subjected to plasma treatment in the same manner as in Example 18. Thereafter, a sample (45) was prepared in the same manner as in Example 18, except that the polyethylene tube having undergone the plasma treatment was dipped into 25 mL of a mixed solution of acetone-methylene chloride (96:4 (volume ratio)) containing only 250 mg of tris-[(3-mercaptopropionyloxy)-ethyl]-isocyanurate (TEMPIC) (manufactured by SC Organic Chemical Co., Ltd.; having three thiol groups in a single molecule).

The obtained sample (45) was evaluated in terms of antithrombotic in the same manner as in Example 1. The result is shown in Table 4. Moreover, the obtained sample (45) was evaluated in terms of surface lubricity and surface lubricity retention in the same manner as in Example 11. The results are shown in Table 4.

### (Comparative example 28)

A polyethylene tube was subjected to plasma treatment in the same manner as in Example 18. Thereafter, a sample (46) was prepared in the same manner as in Example 18, except that the polyethylene tube having undergone the plasma treatment was dipped into 25 mL of a mixed solution of acetone-methylene chloride (96:4 (volume ratio)) containing nothing.

The obtained sample (46) was evaluated in terms of antithrombotic in the same manner as in Example 1. The result is shown in Table 4. Moreover, the obtained sample (46) was evaluated in terms of surface lubricity and surface lubricity retention in the same manner as in Example 11. The results are shown in Table 4.

### (Example 19)

A wire (φ 0.7 mm, length of 150 mm) made of SUS 304 was dipped into 25 mL of a mixed solution of acetone-methylene chloride (96:4 (volume ratio)) containing 250 mg of tris-[(3-mercaptopropionyloxy)-ethyl]-isocyanurate (TEMPIC) (manufactured by SC Organic Chemical Co., Ltd.; having three thiol groups in a single molecule) and 250 mg polyethylene glycol methyl ether methacrylate (manufactured by Sigma-Aldrich Co. LLC., molecular weight of 1,100), dried at 25°C, followed by a reaction for 10 minutes under UV irradiation at room temperature (25°C) to form a hydrophilic layer (thickness:1 µm) on the wire made of SUS304, thereby preparing a sample (47).

The obtained sample (47) was evaluated in terms of antithrombotic in the same manner as in Example 1. The result is shown in Table 4. Moreover, the obtained sample (47) was evaluated in terms of surface lubricity and surface lubricity retention in the same manner as in Example 11. The results are shown in Table 4.

### (Comparative example 29)

A sample (48) was prepared in the same manner as in Example 19, except that the wire (φ 0.7 mm, length of 150 mm) made of SUS 304 was dipped into 25 mL of a mixed solution of acetone-methylene chloride (96:4 (volume ratio)) containing only 250 mg of polyethylene glycol methyl ether methacrylate (manufactured by Sigma-Aldrich Co. LLC., molecular weight of 1,100) in Example 19.

The obtained sample (48) was evaluated in terms of antithrombotic in the same manner as in Example 1. The result is shown in Table 4. Moreover, the obtained sample (48) was evaluated in terms of surface lubricity and surface lubricity retention in the same manner as in Example 11. The results are shown in Table 4.

### (Comparative example 30)

A sample (49) was prepared in the same manner as in Example 19, except that the wire (φ 0.7 mm, length of 150 mm) made of SUS 304 was dipped into 25 mL of a mixed solution of acetone-methylene chloride (96:4 (volume ratio)) containing only 250 mg of tris-[(3-mercaptopropionyloxy)-ethyl]-isocyanurate (TEMPIC) (manufactured by SC Organic Chemical Co., Ltd.; having three thiol groups in a single molecule) in Example 19.

The obtained sample (49) was evaluated in terms of antithrombotic in the same manner as in Example 1. The result is shown in Table 4. Moreover, the obtained sample (49) was evaluated in terms of surface lubricity and surface lubricity retention in the same manner as in Example 11. The results are shown in Table 4.

### (Comparative example 31)

A sample (50) was prepared in the same manner as in Example 19, except that the wire (φ 0.7 mm, length of 150 mm) made of SUS 304 was dipped into 25 mL of a mixed solution of acetone-methylene chloride (96:4 (volume ratio)) containing nothing in Example 19.

The obtained sample (50) was evaluated in terms of antithrombotic in the same manner as in Example 1. The result is shown in Table 4. Moreover, the obtained sample (50) was evaluated in terms of surface lubricity and surface lubricity retention in the same manner as in Example 11. The results are shown in Table 4.

**[Table 4]**

| | Base material | Added compound 1 | Added compound 2 | Treatment method | Value of slide resistance | Slide retention -ability | Platelet count (average) |
|---|---|---|---|---|---|---|---|
| Example 11 | SUS | TEMPIC | PEG-SH | 120°C | ○ | ○ | 0.3 |
| Comparative example 11 | SUS | - | PEG-SH | 120°C | ○ | × | 505 |
| Comparative example 12 | SUS | TEMPIC | - | 120°C | × | × | 610 |
| Comparative example 13 | SUS | - | - | 120°C | × | × | 605 |
| Example 12 | Ni-Ti | TEMPIC | PEG-SH | 120°C | ○ | ○ | 0.3 |
| Comparative example 14 | Ni-Ti | - | PEG-SH | 120°C | ○ | × | 510 |
| Comparative example 15 | Ni-Ti | TEMPIC | - | 120°C | × | × | 600 |
| Comparative example 16 | Ni-Ti | - | - | 120°C | × | × | 610 |
| Example 13 | Polyethylene | TEMPIC | PEG-SH | Plasma | ○ | ○ | 0.3 |
| Comparative example 17 | Polyethylene | - | PEG-SH | Plasma | × | × | 605 |
| Comparative example 18 | Polyethylene | TEMPIC | - | Plasma | × | × | 605 |
| Comparative example 19 | Polyethylene | - | - | Plasma | × | × | 610 |
| Example 14 | Glass | TEMPIC | PEG-SH | Plasma | ○ | ○ | 0.3 |
| Comparative example 20 | Glass | - | PEG-SH | Plasma | × | × | 605 |
| Comparative example 21 | Glass | TEMPIC | - | Plasma | × | × | 600 |
| Comparative example 22 | Glass | - | - | Plasma | × | × | 595 |
| Example 15 | SUS | DPMP | PEG-SH | 120°C | ○ | ○ | 0.3 |
| Comparative example 23 | SUS | DPMP | - | 120°C | × | × | 605 |
| Example 16 | SUS | EGMP-4 | PEG-SH | 120°C | ○ | ○ | 0.3 |
| Comparative example 24 | SUS | EGMP-4 | - | 120°C | × | × | 610 |
| Example 17 | SUS | TEMPIC | PEG-SH2 | 120°C | ○ | ○ | 0.3 |
| Comparative example 25 | SUS | - | PEG-SH2 | 120°C | ○ | × | 510 |
| Example 18 | Polyethylene | TEMPIC | PEG-SH | UV | ○ | ○ | 0.3 |
| Comparative example 26 | Polyethylene | - | PEG-SH | UV | × | × | 610 |
| Comparative example 27 | Polyethylene . | TEMPIC | - | UV | × | × | 595 |
| Comparative example 28 | Polyethylene | - | - | UV | × | × | 600 |
| Example 19 | SUS | TEMPIC | PEG-MA | UV | ○ | ○ | 0.3 |
| Comparative example 29 | SUS | - | PEG-MA | UV | × | × | 600 |
| Comparative example 30 | SUS | TEMPIC | - | UV | × | × | 610 |
| Comparative example 31 | SUS | - | - | UV | × | × | 595 |

As shown in Table 4, the samples of examples having the hydrophilic layer containing the hydrophilic compound and the thiol compound according to the present invention exhibit excellent antithrombotic, compared to the samples of comparative examples having a base material only, a layer formed only of the hydrophilic compound or a layer formed only of the thiol compound. Table 4 also shows that in the samples of Examples 11 to 19 using the hydrophilic compound having polyoxyethylene groups as hydrophilic groups, the value of slide resistance is significantly lowered (that is, surface lubricity is excellent), the value of slide resistance can be kept low, and the hydrophilic layer exhibits excellent durability (that is, surface lubricity maintainability is excellent), compared to the samples of comparative examples having a base material only, a layer formed only of the hydrophilic compound or a layer formed only of the thiol compound.

The present application is based on Japanese Patent Application No. 2011-189728 filed on august 31, 2011, the entire content of which is incorporated herein as reference.

## Claims

1. A medical device comprising:
a base material layer; and
a hydrophilic layer that is supported on at least a portion of the base material layer,
wherein the hydrophilic layer is obtained by forming a crosslinked structure by means of reacting a compound having plural thiol groups in a molecule with a compound having reactive functional groups binding to the thiol group and hydrophilic groups in a molecule, and
the reactive functional groups are thiol groups, alkenyl groups, acryl groups, or methacryl groups.

2. The medical device according to Claim 1,
wherein the hydrophilic groups are polyoxyethylene groups, hydroxyl groups, carboxyl group, amide group, sulfonic acid groups, or salts of these.

3. The medical device according to Claim 1 or 2,
wherein the hydrophilic layer is obtained by coating the base material layer with a mixed solution formed of a single solution that is obtained by dissolving the compound having plural thiol groups in a molecule and the compound having the reactive functional groups and hydrophilic groups in a solvent, and then reacting the thiol groups with the reactive functional groups.
